# EUROPEAN PATENT SPECIFICATION

(11) **EP 1 727 793 B1**
(45) Date of publication and mention of the grant of the patent: **09.04.2008**
(21) Application number: 05707484.1
(22) Date of filing: 16.02.2005
(51) Int. Cl.: C07D 205/04, A61K 31/397

(54) **SUBSTITUTED AZETIDINE COMPOUNDS AS CYCLOOXYGENASE-1 - CYCLOOXYGENASE-2 INHIBITORS, AND THEIR PREPARATION AND USE AS MEDICAMENTS**
SUBSTITUIERTE AZETIDINVERBINDUNGEN ALS CYCLOOXIGENASE-1-CYCLOOXYGENASE-2-INHIBITOREN UND DEREN HERSTELLUNG UND VERWENDUNG ALS MEDIKAMENT
COMPOSES AZETIDINE SUBSTITUES UTILES COMME INHIBITEURS DE CYCLOOXYGENASE-1-CYCLOOXYGENASE-2 ET LEUR PREPARATION ET UTILISATION COMME MEDICAMENTS

(30) Priority: 16.02.2004 ES 200400363; 19.03.2004 US 804505
(43) Date of publication of application: 06.12.2006
(73) Proprietor: LABORATORIOS DEL DR. ESTEVE, S.A., 08041 Barcelona (ES)
(72) Inventor: CUBERES ALTISEN, Rosa, Barcelona (ES); FRIGOLA CONSTANSA, Jordi, Barcelona (ES); ALVAREZ MATHIEU, Ines, 08041 Barcelona (ES)
(74) Representative: Ponti Sales, Adelaida
(86) International application number: PCT/EP2005/001657
(87) International publication number: WO 2005/077896

(56) References cited:
- EP-A- 1 364 949
- WO-A-20/04011421
- US-A- 6 004 948
- US-B1- 6 472 416

## Description

The present invention relates to substituted Azetidine compounds of general formula (I), methods for their preparation, medicaments comprising these compounds as well as their use for the preparation of a medicament for the treatment of humans and animals.

The metabolites of arachidonic acid, such as prostaglandins, lipoxygenases and thromboxane products are produced in a wide variety of tissues and play a key role in many physiological and pathophysiological processes, such as inflammation, pain and cancer.
Prostaglandins, for example, are produced from cell membrane phospholipids via a cascade of enzymes, involving the conversion of arachidonic acid to a common prostaglandin precursor, PGH₂, by the enzyme Cyclooxygenase. Today, two different subtypes of Cyclooxygenase are known, namely Cyclooxygenase-1 (COX-1) and Cyclooxygenase-2 (COX-2).

COX-1, which is not-inducible or modulated by glucocorticoids, is the constitutive cyclooxygenase isoform and is mainly responsible for the synthesis of cytoprotective prostaglandins in the gastrointestinal tract and the synthesis of thromboxane which triggers platelet aggregation in blood platelets. COX-2 is inducible and generally short lived except in the case of certain tumors where it is constitutively activated. COX-2 expression is stimulated in response to endotoxins, cytokines, hormones, growth factors and mitogens. _

Thus, the object of the present invention was to provide novel compounds that are particularly suitable as pharmacologically active substances in medicaments. Preferably these compounds should be suitable for inhibition of the Cyclooxygenase-1 and/or Cyclooxygenase-2 and for the prophylaxis and/or treatment of disorders related to these enzymes.

It has surprisingly been found that the substituted compounds of general formula I given below, stereoisomers thereof, corresponding salts and corresponding solvates show inhibition of Cyclooxgenase-1 and Cyclooxygenase-2.

Thus, in one of its aspects the present invention relates to substituted azetidine compounds of general formula I, 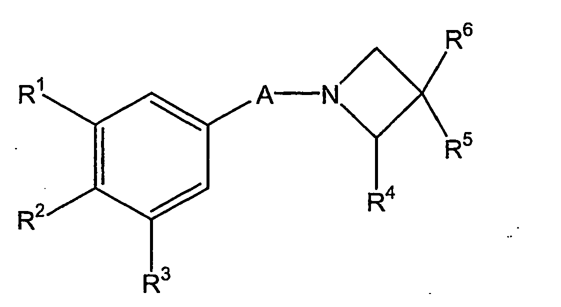 wherein
A represents a -C=O-moiety, a -CH₂-moiety, a -CH₂-C=O-moiety bonded to the azetidine ring via its carbonyl carbon atom, or a -O-C(=O)-moiety bonded to the azetidine ring via its carbonyl carbon atom,
R¹, R³, identical or different, represent a hydrogen atom or a linear or branched, saturated or unsaturated, optionally at least mono-substituted C₁₋₄-aliphatic group,
R² represents a hydrogen atom, a hydroxyl group or a C₁₋₃-alkoxy group,
or R¹ and R² or R² and R³ together form an -O-CH₂-CH₂-chain, which is optionally substituted with one or more methyl groups,
R⁴ represents a hydrogen atom, an optionally at least mono-substituted aryl group, or a linear or branched, saturated or unsaturated aliphatic group, whereby said aliphatic group may be substituted by one or more substituents independently selected from the group consisting of hydroxy, fluorine, chlorine, bromine, branched or unbranched C₁₋₄-alkoxy, branched or unbranched C₁₋₄-perfluoroalkoxy and branched or unbranched C₁₋₄-perfluoroalkyl,
R⁵ represents a hydrogen atom, a halogen atom, a hydroxyl group, a linear or branched, saturated or unsaturated, optionally at least mono-substituted aliphatic group, an -OR⁷-moiety, -an -NH₂-moiety, a -CO-NH₂-moiety, an -NH-CO-R⁸-moiety, an -N(OH)-CO-NH₂-moiety, an -O(CH₂)₁₋₄ONO₂-moiety, an optionally at least mono-substituted aryl group, or a carboxy-group,
R⁶ represent a hydrogen atom, a halogen atom, a hydroxyl group, a linear or branched, saturated or unsaturated, optionally at least mono-substituted aliphatic group, an -OR⁹-moiety, -an -NH₂-moiety, a -CO-NH₂-moiety, an -NH-CO-R¹⁰-moiety, an -N(OH)-CO-NH₂-moiety, an optionally at least mono-substituted aryl group, or a carboxy-group,
R⁷, R⁸, R⁹, R¹⁰, independent from one another, represent a linear or branched, saturated or unsaturated, optionally at least mono-substituted aliphatic group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

It is highly preferred that for the substituted azetidine compounds of general formula I given above one or more of the following provisos (disclaimer) apply, namely
that R¹, R² and R³ do not identically represent a hydrogen atom, and if A represents a -CH₂-moiety, then at least two of the residues R¹, R² and R³ do not identically represent a hydrogen atom,
that if A represents a -(C=O)-moiety, R⁴, represents a hydrogen atom and one of the residues R⁵ and R⁶ represents a hydrogen atom, then the other one of these residues R⁵ and R⁶ does not represent an -NH₂-moiety, a -CONH₂-moiety or a methyl group, which is substituted by an -NH₂-moiety or an optionally substituted azaheterocycle, and
that if A represents a -(C=O)-moiety, a -CH₂-C=O-moiety bonded to the azetidine ring via its carbonyl atom, or a -O-C(=O)-moiety bonded to the azetidine ring via its carbonyl carbon atom and one of the residues R⁵ and R⁶ represents a hydrogen atom or a linear or branched, saturated or unsaturated, optionally at least mono-substituted aliphatic group, then the other one of these residues R⁵ and R⁶ does not represent an -NH₂- or -carboxy -moiety,

If any of the afore mentioned substituents represents an aliphatic group, which is substituted by one or more, e.g. 1, 2, 3, 4 or 5, substituents, these substituents may, independent from one another, preferably be selected from the group consisting of hydroxy, fluorine, chlorine, bromine, branched or unbranched C₁-₄-alkoxy, branched or unbranched C₁₋₄-perfluoroalkoxy, branched or unbranched C₁₋₄-perfluoroalkyl, more preferably be selected from the group consisting of hydroxy, F, Cl, Br, methoxy, ethoxy and CF₃.

Preferred linear or branched, saturated or unsaturated aliphatic groups, which may be substituted by one or more substituents, may preferably be selected from the group consisting of methyl, ethyl, n-propyl, isopropyl, n-butyl, iso-butyl, sec-butyl, tert-butyl, n-pentyl, n-hexyl, n-heptyl, n-octyl, n-nonyl, n-decyl, vinyl, ethinyl, propenyl, propinyl, butenyl and butinyl:

If any of the afore mentioned substituents represents an aryl group, which is substituted by one or more, e.g. 1, 2, 3, 4 or 5, substituents, these substituents may, independent from one another, preferably be selected from the group consisting of hydroxy, fluorine, chlorine, bromine, branched or unbranched C₁₋₄-alkyl, branched or unbranched C₂₋₄-alkenyl, branched or unbranched C₂₋₄-alkinyl, branched or unbranched C₁₋₄-alkoxy, branched or unbranched C₁₋₄-perfluoroalkoxy, branched or unbranched C₁₋₄-perfluoroalkyl, more preferably be selected from the group consisting of hydroxy, F, Cl, Br, methyl, ethyl, n-propyl, iso-propyl, tert.-Butyl, n-Butyl, sec-Butyl, methoxy, ethoxy and CF₃.

Preferred aryl groups, which may optionally be at least mono-substituted, are phenyl and naphthyl.

Preferred are compounds of general formula I given above, wherein R¹ and R³, identical or different, represent a hydrogen atom or a linear or branched C₁₋₄-alkyl group, preferably R¹ and R³ are identical and represent an unsubstituted C₁₋₄-alkyl group, more preferably R¹ and R³ are identical and represent a C₃₋₄ alkyl group, most preferably R¹ and R³ are identical and represent an iso-propyl group or a tert.-Butyl group and R², R⁴-R¹⁰ and A have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Also preferred are compounds of general formula I given above, wherein R² represents a hydrogen atom, a hydroxyl group or a methoxy group, and R¹, R³-R¹⁰ and A have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Furthermore, compounds of general formula I are preferred, in which R⁴ represents a hydrogen atom, an optionally at least mono-substituted phenyl group, or a linear or branched, saturated or unsaturated C₁₋₆ aliphatic group, whereby said aliphatic group may be substituted with one or more substituents independently selected from the group consisting of hydroxy, fluorine, chlorine, bromine, branched or unbranched C₁₋₄-alkoxy, branched or unbranched C₁₋₄-perfluoroalkoxy and branched or unbranched C₁₋₄-perfluoroalkyl, more preferably R⁴ a hydrogen atom, a methyl group or an unsubstituted phenyl group and R¹-R³, R⁵-R¹⁰ and A have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Preference is also given to compounds of general formula I given above, in which R⁵ represents a hydrogen atom, a halogen atom, a hydroxyl group, a linear or branched, saturated or unsaturated, optionally at least mono-substituted C₁₋₆ aliphatic group, an -NH₂-moiety, a -CO-NH₂-moiety, an -NH-CO-R⁸-moiety, an -N(OH)-CO-NH₂-moiety, an -O(CH₂)₄-ONO₂-moiety, an optionally at least mono-substituted phenyl group, or a carboxy-group, preferably a hydrogen atom, a bromine atom, a hydroxyl group, an -NH₂-moiety, a -CO-NH₂-moiety, an -NH-CO-R⁸-moiety, an -N(OH)-CO-NH₂-moiety, -O(CH₂)₄-ONO₂-moiety, an unsubstituted phenyl group, or a carboxy-group, and R¹-R⁴, R⁶-R¹⁰ and A have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Also preferred are compounds of general formula I, in which R⁶ represents a hydrogen atom, a halogen atom, a hydroxyl group, a linear or branched, saturated or unsaturated, optionally at least mono-substituted C₁₋₆ aliphatic group, an -NH₂-moiety, a -CO-NH₂-moiety, an -NH-CO-R⁸-moiety, an -N(OH)-CO-NH₂-moiety, an optionally at least mono-substituted phenyl group, or a carboxy-group, preferably a hydrogen atom, a hydroxyl group or a methyl group, and R¹-R⁵, R⁷-R¹⁰ and A have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Preferred are also compounds of general formula I given above, in which R⁷, R⁸, R⁹, R¹⁰, independent from one another, represent a linear or branched, saturated or unsaturated, optionally at least mono-substituted C₁₋₆ aliphatic group, preferably a linear or branched C₁₋₆ alkyl group, more preferably a methyl group or an ethyl group, and R¹-R⁶ and A have the meaning given above, optionally in form of one of the stereoisomers, preferably enantiomers or diastereomers, a racemate or in form of a mixture of.at-least two of the stereoisomers, preferably enantiomers and/or diastereomers, in any mixing ratio, or a corresponding salt thereof, or a corresponding solvate thereof.

Particularly preferred are substituted azetidine compounds of general formula 1, 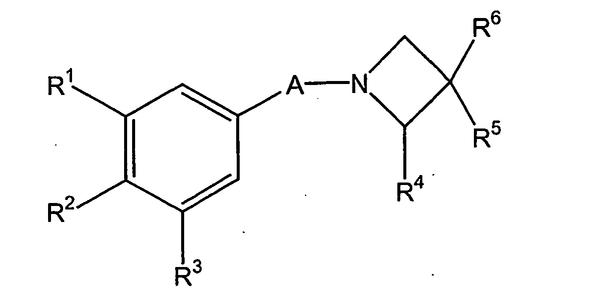 wherein
A represents a -C=O-moiety, a -CH₂-moiety, a -CH₂-C=O-moiety bonded to the azetidine ring via its carbonyl carbon atom, or a -O-C(=O)-moiety bonded to the azetidine ring via its carbonyl carbon atom,
R¹, R³ both represent an iso-propyl group or a tert.-butyl group,
R² represents a hydrogen atom, a hydroxyl group or a methoxy group,
or R¹ and R² or R² and R³ together form an -O-CH₂-C(CH₃)₂-chain, whereby the oxygen atom of said chain is bonded to the 4-position of the phenyl ring,
R⁴ represents a hydrogen atom, a methyl group or an unsubstituted phenyl group,
R⁵ represents a bromine atom, a hydroxyl group, -an -NH₂-moiety, a -CO-NH₂-moiety, an -NH-CO-CF₃-moiety, an -N(OH)-CO-NH₂-moiety, an -O(CH₂)₄ONO₂-moiety, an unsubstituted phenyl group, or a carboxy-group,
R⁶ represent-a hydrogen atom, a methyl group or a hydroxyl group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

Most particularly preferred are compounds of general formula I selected from the group consisting of
[1] (3,5-di-tert-butyl-4-hydroxy-phenyl)-(3-hydroxy-azetidin-1-yl)methanone;
[2] (3,5-di-tert-butyl-phenyly(3-hydroxy-azetidin-1 -yi)-methanone;
[3] (3,5-di-tert-butyl-4-hydroxy-phenyl)-(3-hydroxy-3-methyl-azetidin-1-yl)-methanone;
[4] (3,5-di-tert-butyl-4-hydroxy-phenyl)-(3-hydroxy-2-methyl-azetidin-1-yl)-methanone;
[7] (3-Bromo-azetidin-1 -yl)-(3,5-di-tert-butyl-4-hydroxy-phenyl)-methanone ;
[9] (3,5-di-tert-butyl-4-methoxy-phenyl)-(3-hydroxy-azetidin-1-yl)-methanone;
[10] (3-hydroxy-azetidin-1-yl)-(4-hydroxy-3,5-diisopropyl-phenyl)-methanone;
[11] (3,5-di-tert-butyl-phenyl)-[3-(4-nitrooxy-butoxy)-azetidin-1-yl]-methanone;
[12] (3,5-di-tert-butyl-4-hydroxy-phenyl)-(3-hydroxy-2-phenyl-azetidin-1-yl)-methanone;
[13] (3,5-di-tert-butyl-4-hydroxy-phenyl)-(3-hydroxy-3-phenyl-azetidin-1-yl)-methanone;
[14] - (7-tert-butyl-3,3-dimethyl-2,3-dihydro-benzofuran-5-yl)-(3-hydroxy-azetidin-1-yl)-methanone;
[15] [1-(3,5-di-tert-butyl-4-hydroxy-benzyl)-azetidin-3-yl]-N-hydroxy-urea;
[16] N-[1-(3,5-di-tert-butyl-4.-hydroxy-benzoyl)- (2S,3R)-2-methyl-azetidin-3-yl]-2,2,2-trifluoro-acetamide;
[17] 1-(3,5-di-tert-butyl-4.-hydroxy-benzyl)-azetidin-3-ol;
[18] 2-(3,5-di-tert-butyl-4-hydroxy-phenyl)-1-(3-hydroxy-azetidin-1-yl)-ethanone;
[19] (3-hydroxy-azetidine-1-carboxylic acid)-3,5-di-tert-butyl-phenyl ester optionally in form of a corresponding salt or a corresponding solvate.

In another aspect the present invention relates to a process for the preparation of the inventive substituted azetidine compounds of general formula I given above, according to which at least one compound of general formula II, 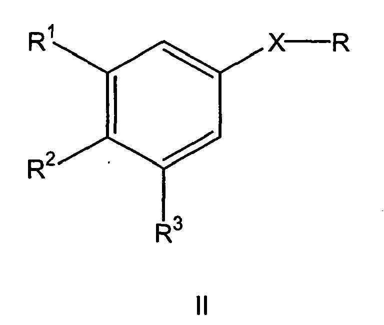 wherein R¹-R³ have the meaning given above, X represents a bond or an -(CH₂)-moiety and R represents a carboxy group or an activated carbonyl group, is reacted with at least one compound of general formula III, 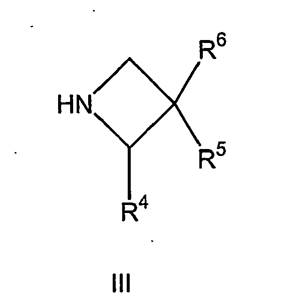 optionally in the form of a corresponding salt, wherein R⁴-R⁶ have the meaning given above, to yield a compound of general formula I given above, wherein R¹ to R⁶ have the meaning given above and A represents a -(C=O)-moiety or a -(CH₂)-CO-moiety, which is optionally purified and/or optionally isolated,
and optionally at least one compound of general formula l, wherein A represents a - (C=O)-moiety is reduced to yield at least one compound of general formula I, wherein R¹-R⁶ have the meaning given above and A represents a -(CH₂)-moiety, which is optionally purified and/or optionally isolated,
or at least one compound of general formula IV, 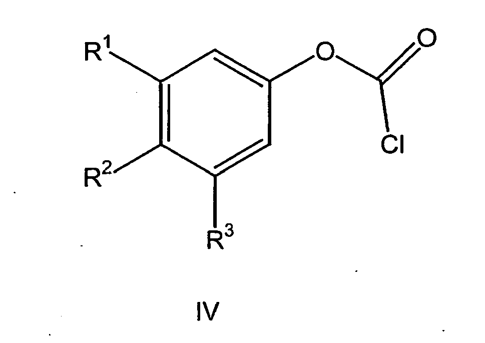 wherein R¹-R³ have the meaning given above, is reacted with at least one compound of general formula III given above, to yield at least one compound of general formula I, wherein R¹-R⁶ have the meaning given above and A represents an O-(C=O)-moiety, and said compound is optionally purified and/or optionally isolated.

Compounds of general formula (II) may be prepared by conventional methods known to those skilled in the art.

The reaction of compounds of general formula (II) and general formula (III) may also be carried out according to conventional methods well known to those skilled in the art. The compounds of general formula II may either be used in form of the free carboxylic acid, i.e. R represents a -COOH group, or in form of an activated carbonyl group. Suitable activating groups and methods for activation are well-known to those skilled in the art, e.g. activation with N,N-Dicyclohexylcarbodiimide or other coupling reagents.

The reaction between compounds of of general formula II and compounds of general formula III is preferably carried out in a suitable reaction medium such as diethyl ether, tetrahydrofuran, dioxane, dimethoxyethane and the like. Reaction temperatures and reaction times may vary over a broad range. The temperature is preferably kept in the range of ambient temperature, i.e. approximately 25 °C and the boiling point of the reaction medium. Suitable reaction times may vary over a broad range, i.e. from a few minutes to several hours.

Other compounds of general formula II, wherein R represents an activated carbonyl group include but are not limited to the acide chlorides, anhydrides, mixed anhydrides, alkyl esters, preferably C₁₋₄ alkyl esters or activated ester, e.g. p-nitrophenyl esters.

If the activated compound of general formula II is an acid chloride it is preferably prepared by conventional methods well known to those skilled in the art, e.g. by reaction of the respective compound of general formula II in form of the free carboxylic acid with thionyl chloride or oxalyl chloride, whereby said chlorinating agent may also be used as the reaction medium, optionally in a mixture with at least one other reaction medium. Other suitable reaction media include but are not limited thereto hydrocarbons such as benzene, toluene or xylene, halogenated hydrocarbons such as mothylenechloride, chloroform or carbon tetrachloride, or ethers such as diethyl ether, dioxane, tetrahydrofuran or dimethoxyethane or mixtures of two or more of these afore mentioned compounds. Suitable reaction temperatures and reaction times may vary over a broad range, for example, from 0°C to the boling point of the reaction medium and from several minutes to several hours.

The reaction of an acid chloride of general formula II with an azetidine compound of general formula III is preferably carried out in the presence of inorganic base such as sodium hydroxide, potassium hydroxide, sodium carbonate, potassium carbonate and the like and/or in the presence of an organic base such as triethyl amine, pyridine and the like in a suitable reaction medium such as a hydrocarbons like benzene, toluene or xylene, halogenated hydrocarbons like methylenchloride, chloroform or carbon tetrachloride, or ethers such as diethyl ether, dioxane, tetrahydrofuran or dimethoxyethane or mixtures of at least two or more of the afore mentioned compounds. Said reaction may also be carried out in a reaction medium based on one or more of the afore mentioned compounds and water in a biphasic system.

Those skilled in the art understand that if the compound of general formula II is present in the form of an acid chloride and compound of general formula III is substituted with one or two hydroxyl groups in the 3-position of the azetidine ring, then reaction may also take place between the acid chloride and said alcohol group(s). In this case, the compound of general formula I may be obtained via selective hydrolysis of the respective ester by reaction with a suitable base, preferably lithium hydroxide, in a water-based reaction medium, whereby the reaction medium may comprise conventional organic solvents, which are partially or totally miscible with the aqueous phase, such as methanol, ethanol, propanol and the like or a suitable ether such as tetrahydrofuran, dioxane or dimethoxyethane. Reaction temperature and reaction time may vary over a broad range, preferably from -20°C to ambient temperature, i.e. approximately 25 °C and from several hours to several days.

If the activated derivative of general formula is a mixed anhydride said compound may preferably be prepared by reaction of the corresponding free acid with an alkyl chloroformiate or an aryl chloroformiate compound, preferably in the presence of a base such as triethylamine or pyridine in a suitable reaction medium.

The compounds of general formula I, wherein A represents a -(C=O)-moiety, may be reduced to the corresponding compound of geneal formula I, wherein A represents a -(CH₂)-moiety, using at least one suitable reducing agent known to those skilled in the art. A preferred reducing agent is lithium aliuminium hydride. Those skilled in the art understand that if the respective compound of general formula I, wherein A represents a -(C=O)-moiety contains one or more further groups, which are suceptible to reduction, these will also be reduced and suitable steps of protecting these groups may be required.

The reduction is preferably carried out in a suitable reaction medium such as ether, preferably diethyl ether, tetrahydrofurane, dioxane or dimethoxyethane. The reaction temperature and reaction time may vary over a broad range, e.g. from ambient temperature, i.e. approximately 25 °C to the boiling point of the reaction medium and from several minutes to several hours.

The compounds of general formula IV may preferably be prepared from compounds of general formula V, 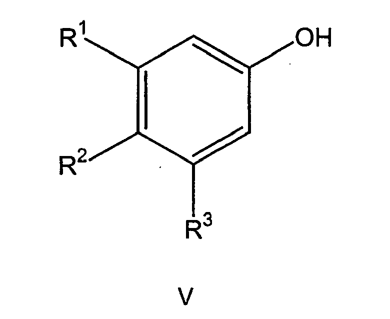 wherein R¹-R³ have the meaning given above, by reaction with diphosgene in an anhydrous solvent such as ether, preferably diethyl ether, tetrahydrofuran, dioxane, dimethoxyethan and the like. Reaction temperatures may vary over a broad range, whereby preferred temperatures range from -20°C to ambient temperature, i.e. approximately 25 °C and suitable reaction times vary from several minutes to several hours.

The reaction of compounds of general formula IV with compounds of general formula III may preferably be carried out in the presence of an organic base such triethylamine, pyridine and the like in a suitable reaction medium such as hydrocarbons like benzene, toluene, xylene, halogenated hydrocarbons like methylene chloride, chloroform, or carbontetrachloride, ethers like diethyl ether, tetrahhydrofuran or dimethoxyethan or mixtures of at least two of these afore mentioned solvents. Reaction temperatures and reaction times may vary over a broad range, preferably from 0°C to the boiling point of the reaction medium and from several minutes to several hours.

The substituted azetidine compounds of general formula III may be prepared by conventional methods described in the prior art, for example in V.R. Gaertner, J. Org. Chem. ,1967, 32, 2972; A.G. Anderson et al., J. Org. Chem., 1972, 37, 3953; N.H. Cromwell et al., Chem. Rev., 1979, 79, 331-358; D. Nisato et al., J. Heterocyclic Chem.; 1985, 22, 961-963; A.P. Kozikowski et al., Synlett, 1991, 783-784; J. Frigola et al., J. Med. Chem., 1993, 36, 801-810; J. Frigola et al., J. Med. Chem., 1994, 37, 4195-4210; J. Frigola et al., J. Med. Chem., 1995, 38, 1203-1215; M. Poch et al., Tetrahedron Letters, 1993, 34 (48), 7781-7784; M. Poch et al., Tetrahedron Letters, 1991, 32 (47), 6935-6938; T. Toda et al., Heterocycles, 1992, 33,-511-514; R.H. Higgins et al., J. Org. Chem., 1994, 59, 2172-2178; J. Barluenga et al., J. Org. Chem., 1997, 62, 5974-5977; US-5073646 and references cited therein. The respective parts of the description are hereby enclosed by reference and form part of the present disclosure.

In a further aspect the present invention also provides a process for the preparation of salts of substituted azetidine compounds of general formula (I), or stereoisomers thereof, wherein at least one compound of general formula (I) having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of substituted azetidine compounds of general formula (I), or stereoisomers thereof, wherein at least one compound of general formula (I) having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or unbranched C₁₋₄-alkyl-radical. Suitable reaction media are, for example, any of the ones given above.

Solvates, preferably hydrates, of the substituted azetidine compounds of general formula (I), of corresponding stereoisomers, or of corresponding salts thereof may also be obtained by standard procedures known to those skilled in the art.

The purification and isolation of the inventive substituted azetidine compounds of general formula (I), of a corresponding stereoisomer, or salt, or solvate or any intermediate thereof may, if required, be carried out by conventional methods known to those skilled in the art, e.g. chromatographic methods or recrystallization.

If the substituted azetidine compounds of general formula (I) themselves are obtained in form of a mixture of stereoisomers, particularly enantiomers or diastereomers, said mixtures may be separated by standard procedures known to those skilled in the art, e.g. chromatographic methods or fractunalized crystallization with chiral reagents. It is also possible to obtain pure stereoisomers via stereoselective synthesis.

The substituted azetidine compounds of general formula (I), their stereoisomers, corresponding salts thereof and corresponding solvates are toxicologically acceptable and are therefore suitable as pharmaceutical active substances for the preparation of medicaments.

It has surprisingly been found that the substituted compounds of general formula I given above, stereoisomers thereof, corresponding salts and corresponding solvates show inhibition of Cyclooxgenase-1 and/or Cyclooxygenase-2.

Thus, in another aspect the present invention relates to a medicament comprising at least one substituted azetidine compound of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof and optionally one or more pharmaceutically acceptable excipients.

Preferably the medicament of the present invention is suitable for the inhibition of Cyclooxygenase-1, for the prophylaxis and/or treatment of Cyclooxygenase-1 related disorders, for the inhibition of Cyclooxgenase-2 and/or for the prophylaxis and/or treatment of Cyclooxygenase-2 related disorders.

Particularly preferably the medicament of the present invention is suitable for the prophylaxis and/or treatment of pain, for the prophylaxis and/or treatment of inflammation and/or for the prophylaxis and/or treatment of inflammation related disorders, whereby said inflammation-related disorders may preferably be selected from the group consisting of arthritis, rheumatoid arthritis, spondyloarthropathies, gouty arthritis, osteoarthritis, systemic lupus erythematosus, juvenile arthritis, rheumatic fever, symptoms associated with influenza or other viral infections, common cold, lower back pain, neck pain, dysmenorrhea, headache, toothache, sprains, strains, myositis, neuralgia, synovitis, gout, ankylosing spondylitis, bursitis, edema, inflammations following dental procedures, inflammations following dental procedures, vascular diseases, migraine headaches, periarteritis nodosa, thyroiditis, aplastic anemia, Hodkin's disease, sclerodoma, type I diabetes, myasthenia gravis, sarcoidosis, nephrotic syndrome, Behcet's syndrome, polymyositis, gingivitis, hypersensivity, conjunctivitis, swelling ocurring after injury and myocardia ischemia, for the prophylaxis and/or treatment of asthma, for the prophylaxis and/or treatment of bronchitis, for the prophylaxis and/or treatment of tendinitis, for the prophylaxis and/or treatment of bursitis, for the prophylaxis and/or treatment of skin related conditions, whereby said skin related conditions may preferably be selected from the group consisting of psoriasis, eczema, burns and dermatitis, for the prophylaxis and/or treatment of gastrointestinal disorders, whereby said gastrointestinal disorders may preferably be selected from the group consisting of inflammatory bowel disease, Crohn's disease, gastritis, irritable bowel syndrome and ulcerative colitis, or for treatment of fever, or for the prophylaxis and/or treatment of cancer or a cancer-related disorders, whereby said cancer or related disorder may preferably be selected from the group consisting of brain cancer, bone cancer, epithelial cell-derived neoplasia (epithelial carcinoma), basal cell carcinoma, adenocarcinoma, gastrointestinal cancer, lip cancer, colon cancer, liver cancer, bladder cancer, pancreas cancer, ovary cancer, cervical cancer, lung cancer, breast cancer, skin cancer, squamous cell cancer, prostate cancer, renal cell carcinoma and other known cancers that effect epithelial cells throughout the body, for the prophylaxis and/or treatment of polyps, for the prophylaxis and/or treatment of angiogenesis mediated disorders, preferably selected from the group consisting of metastasis, corneal graft rejection, ocular neovascularization, retinal neovascularisation, diabethic retinopathy, retrolental fibroplasia, neovascular glaucoma, gastric ulcer, infantile hemaginomas, angiofibroma of the nasopharynx, avascular necrosis of the bone and endometriosis.

Most particularly preferred the medicament of the present invention is suitable for the prophylaxis and/or treatment of pain, for the prophylaxis and/or treatment of inflammation and/or for the prophylaxis and/or treatment of inflammation related disorders, whereby said inflammation-related disorders may preferably be selected from the group consisting of arthritis, rheumatoid arthritis, spondyloarthropathies, gouty arthritis, osteoarthritis, systemic lupus erythematosus, juvenile arthritis, rheumatic fever, symptoms associated with influenza or other viral infections, common cold, lower back pain, neck pain, dysmenorrhea, headache, toothache, sprains, strains, myositis, neuralgia, synovitis, gout, ankylosing spondylitis, bursitis, edema, inflammations following dental procedures, inflammations following dental procedures, vascular diseases, migraine headaches, periarteritis nodosa, thyroiditis, aplastic anemia, Hodkin's disease, sclerodoma, type I diabetes, myasthenia gravis, sarcoidosis, nephrotic syndrome, Behcet's syndrome, polymyositis, gingivitis, hypersensivity, conjunctivitis, swelling ocurring after injury and myocardia ischemia.

In yet another aspect the present invention relates to the use of at least one substituted azetidine of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, optionally in combination with one or more pharmaceutically acceptable excipients, for the preparation of a medicament for the inhibition of Cyclooxygenase-1, for the prophylaxis and/or treatment of Cyclooxygenase-1 related disorders, for the inhibition of Cyclooxgenase-2 and/or for the prophylaxis and/or treatment of Cyclooxygenase-2 related disorders.

The use of at least one substituted azetidine compound of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, optionally in combination with one or more pharmaceutically acceptable excipients, for the preparation of a medicament for the prophylaxis and/or treatment of pain, for the prophylaxis and/or treatment of inflammation and/or for the prophylaxis and/or treatment of inflammation related disorders, whereby said inflammation-related disorders may preferably be selected from the group consisting of arthritis, rheumatoid arthritis, spondyloarthropathies, gouty arthritis, osteoarthritis, systemic lupus erythematosus, juvenile arthritis, rheumatic fever, symptoms associated with influenza or other viral infections, common cold, lower back pain, neck pain, dysmenorrhea, headache, toothache, sprains, strains, myositis, neuralgia; synovitis, gout, ankylosing spondylitis, bursitis, edema, inflammations following dental procedures, inflammations following dental procedures, vascular diseases, migraine headaches, periarteritis nodosa, thyroiditis, aplastic anemia, Hodkin's disease, sclerodoma, type I diabetes, myasthenia gravis, sarcoidosis, nephrotic syndrome, Behcet's syndrome, polymyositis, gingivitis, hypersensivity, conjunctivitis, swelling ocurring after injury and myocardia ischemia, for the prophylaxis and/or treatment of asthma, for the prophylaxis and/or treatment of bronchitis, for the prophylaxis and/or treatment of tendinitis, for the prophylaxis and/or treatment of bursitis, for the prophylaxis and/or treatment of skin related conditions, whereby said skin related conditions may preferably be selected from the group consisting of psoriasis, eczema, burns and dermatitis, for the prophylaxis and/or treatment of gastrointestinal disorders, whereby said gastrointestinal disorders may preferably be selected from the group consisting of inflammatory bowel disease, Crohn's disease, gastritis, irritable bowel syndrome and ulcerative colitis, or for treatment of fever, or for the -prophylaxis and/or treatment of cancer or a cancer-related disorders, whereby said cancer or related disorder may preferably be selected from the group consisting of brain cancer, bone cancer, epithelial cell-derived neoplasia (epithelial carcinoma), basal cell carcinoma, adenocarcinoma, gastrointestinal cancer, lip cancer, colon cancer, liver cancer, bladder cancer, pancreas cancer, ovary cancer, cervical cancer, lung cancer, breast cancer, skin cancer, squamous cell cancer, prostate cancer, renal cell carcinoma and other known cancers that effect epithelial cells throughout the body, for the prophylaxis and/or treatment of polyps, for the prophylaxis and/or treatment of angiogenesis mediated disorders, preferably selected from the group consisting of metastasis, corneal graft rejection, ocular neovascularization, retinal neovascularisation, diabethic retinopathy, retrolental fibroplasia, neovascular glaucoma, gastric ulcer, infantile hemaginomas, angiofibroma of the nasopharynx, avascular necrosis of the bone and endometriosis is particularly preffered.

The use of at least one substituted azetidine compound of general formula I given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof, optionally in combination with one or more pharmaceutically acceptable excipients, for the preparation of a medicament for the prophylaxis and/or treatment of pain, for the prophylaxis and/or treatment of inflammation and/or for the prophylaxis and/or treatment of inflammation related disorders, whereby said inflammation-related disorders may preferably be selected from the group consisting of arthritis, rheumatoid arthritis, spondyloarthropathies, gouty arthritis, osteoarthritis, systemic lupus erythematosus, juvenile arthritis, rheumatic fever, symptoms associated with influenza or other viral infections, common cold, lower back pain, neck pain, dysmenorrhea, headache, toothache, sprains, strains, myositis, neuralgia, synovitis, gout, ankylosing spondylitis, bursitis, edema, inflammations following dental procedures, inflammations following dental procedures, vascular diseases, migraine headaches, periarteritis nodosa, thyroiditis, aplastic anemia, Hodkin's disease, sclerodoma, type I diabetes, myasthenia gravis, sarcoidosis, nephrotic syndrome, Behcet's syndrome, polymyositis, gingivitis, hypersensivity, conjunctivitis, swelling ocurring after injury and myocardia ischemia is most particularly preferred.

The medicament according to the present invention may be in any form suitable for the application to humans and/or animals, preferably humans including infants, children and adults and can be produced by standard procedures known to those skilled in the art. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release.

The liquid oral forms for administration may also contain suitable additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

### Pharmacological Methods:

### I. Cox-1/Cox-2 enzyme assay:

The Cox-1/Cox-2 enzyme assay for the inventive azetidine compounds is carried out according to the following description:

Approximately 390 units of the Cox-I or Cox-2 enzyme (Cayman Chemical, Ann Habor, Michigan, U.S.A., catalogue number 60100 and 60120 respectively are suspended in 200-300 µl of Tris HCl (100mM), hematin (1 mM) and phenol (2mM) buffer, and 200 ml epinephrine (5.8 mM) as chromogen. The compounds, which are to be tested, are solved in dimethylformamide or 0.1 N sodium hydroxide, at a volume of 60p1. The total volume of each enzyme reaction is 0.6 ml. It is taken care that the vehicle concentration in the reaction mixture does not exceed 5 % (volume/volume). The reaction medium is incubated for 4 minutes at 37 °C, and then 100 µl of 0.5 mM arachidonic acid are added as substrate.
Immediately after the addition of the substrate, the slope corresponding to the optical density increase at 480 nm is recorded for 1 minute. The reaction is monitored with a Hewlett-Packard 8452A spectrophotometer.

### II. Determination of Cox-1- and Cox-2-activity in human whole blood

The Cox-1- and Cox-2-activity in human whole blood is determined according to a modification of the method described in the publication of C. Brideau et al., "A human whole blood assay for clinical evaluation of biochemical efficacy of cyclooxygenase inhibitors", Inflamm Res 1996; 45: 68-74. The respective part of the description is hereby incorporated by reference and forms part of the present disclosure.

For Cox-1 activity determination, fresh human blood is distributed in a volume of 0.5 ml to silicone Eppendorf tubes, into which 2µl of the compound to be tested or dimethylsulfoxide (DMSO) at a final concentration of 0.4% (volume/volume). The tubes are inverted for homogenization and incubated for 1 hour at 37 °C under slight stirring. Afterwards they are centrifuged for 15 min at 10500g and 100 µl of serum is collected. Proteins are precipitated by the addition of 400µl of methanol to each tube, tubes were centrifuged for 10 minutes at 5000g and 300 µl of supernatant is aspirated and then dried under nitrogen atmosphere.
Tromboxane B2 (TXB2) concentration is quantified using an assay kit (Caymann Chemical, Ann Arbor, Michigan, U.S.A., Catalogue number 519031) after reconstitution into 300 µl assay buffer supplied in the assay kit.

For Cox-2 activity determination, tubes are prepared with 2 µl of the compound to be tested or DMSO, 12 µg/ml of aspirine, 9 µl of 1 % (weight/volume) sodium heparine and 0.5 ml of fresh human blood. Samples are pre-incubated at 37°C during 15 minutes and E. coli 0111:B4 LPS (from Sigma Chemical, St. Louis, Missouri, U.S.A., catalogue number L-3012) are added for incubation during 24 h at 37°C. The samples are centrifuged for 100 µl of plasma collection. 400 µl of Ethyl acetate and 1 % (volume/volume) methanol are added to each sample and the tubes are stirred and centrifuged for 10 minutes at 5000g at 4°C. 300µl of supernatant are aspirated and evaporated under nitrogen atmosphere. Prostaglandin E2 concentration is quantified using an assay kit from Cayman Chemical, Ann-Arbor, Michigan, U.S.A. (Catalogue number: 514010) after reconstitution into 300 µl assay buffer supplied in the assay kit.

### III: Analgesia Test in rats

The inventive compounds are tested for analgesic activity as described above is carried out as described in the publication of K. Hargreaves et al., Pain, 32, 77-88, (1988). The respective part of the description is hereby incorporated by reference and forms part of the disclosure.

The rats are transferred to the experimentation laboratory, where they remain in groups of 5, in makrolon cages with a barred floor to avoid coprophagy. At the beginning of the experiment, water and food were removed, and animals were adequately weighed and marked.

Each rat receives via subplantar injection 0.1 ml of sterile saline solution into the left hind paw, followed by 0.1 ml of a 2 % (weight/volume) carrageenan suspension in sterile saline solution into the right hind paw.

Two hours after the subplantar injections of carrageenan and vehicle, each rat receives by oral route the compounds to be tested, suspended in 5 % (weight/volume) gum arabic, administered at 10 ml per kg of body weight.
Two hours after the administration of the compounds to be tested, the values for analgesic activity are determined. To this purpose, the rats are transferred to the methacrylate chambers of an analgesimeter provided with a glass floor.
Once the acclimatisation period in the chambers is over (i.e. after 5 minutes) an infra-red beam lamp capable of producing a thermal stimulus, is placed below the rat's paws.
The thermal stimulus, previously calibrated at 10 Amperes, is applied to each of the hind paws with at least 1 minute intervals. The response of the rats to pain consists of raising the paw, thus avoiding contact with the floor. Simultaneously, the infra-red light is automatically turned down, and the digital display of the device shows the latency time in seconds. The rats are tested once only to avoid possible learning behaviour.

### IV. Test for activity against Edema in rats

The test for activity against edema is carried out as described in the publication of Winter et al., Proc. Soc. Exp. Biol. Med. 111, 544-547, (1962). The respective part of the description is hereby incorporated by reference and forms part of the present disclosure.

At the beginning of each experimental session the rats are deprived of food, and kept in cages within groups of 5 animals, the cages being fitted with a grating on the floor to prevent coprophagy. After a period of 24 hours without food, the animals are marked in a suitable way, weighed and hydrated via oral administration of 30 ml/ kg body weight of tap water. Half an hour after the hydratisation, the compounds to be tested are administered via oral route, in an amount of 10 ml/ kg body weight as a suspension in gum arabic at 5 % (weight/volume) . One hour after administration of the compounds the animals receive 0.1 ml of the inflammation causing agent (carrageenin 1 % weight/volume, in sterile solution), injected via subplantar route into the right hind paw of the rats. Immediately after the carrageenin injection the volume of the injected paw is determined using a plethysmometer. The readings are expressed in ml. Readings of the volume of the paw are taken every hour after administration of the carrageenin for 7 hours.

### V: Test for antiarthritic activity in rats

The test for antiarthritic activity is carried out as described in the publication of Anderson et al., J. Clin. Invest. 97, 11, 2672-2679, (1996).The respective part of the description is hereby incorporated by reference and forms part of the present disclosure.

On day 0 of the experiment, the volumes of the contralateral paws to those injected, i.e..left hind paws, are measured by means of a pletismometer, the readings of which are expressed in ml.
Next, each rat is injected the adjuvant, consisting of 1 mg of *Mycobacterium butyricum* suspended in 0.1 ml of mineral oil, via subplantar route in the right hind paw.
Approximately every day, and for 15 days after the injection of adjuvant, the volumes of non-injected hind paws (contralateral) are measured again. On day 15, those rats of which the contralateral paws show an increase of at least 0.42 ml compared to the day of the adjuvant injection are selected, discarding those animals with lower volume increases, since their inflammation is not considered to have the adequate magnitude.
On day 15 of the experiment, daily administration of the compounds to be tested via oral route is started, and on each day, the volumes of non-injected hind paws is recorded.
On Day 25 of the experiment, the body weights and non-injected hind paws (contralateral) volumes are measured for the last time, and these values were used to determine the activities.

### VI: PGE2 production in rat inflammatory exudate and gastric mucosa

The PGE2 production in rat inflammatory exudate and gastric mucosa is carried out as described in the publication of O Tofanetti et al., "Effect of nimesulide on cyclooxygenase activity in rat gastric mucosa and inflammatory exudate", Med Sci Res 17, 745-746 (1989). The respective part of the description is hereby incorporated by reference and forms part of the present disclosure.

For this text 6 male wistar rats (Interfauna-St Feliú de Codines, Barcelona) of approximately 200 g (each approximately 6 weeks of age) are used. The compounds to be tested are administered via oral route in gum Arabic at 5% (weight/volume) at a rate of 10ml/kg.

One hour after administration of the compounds and under halothane anaesthesia, each rat is implanted subcutaneously in the interescapular area a 40X15X5 mm polyester sponge, soaked in a 0.5% suspension of carrageenan. Rats are sacrificed 6 hours after the implant, the sponges are removed and squeezed. The exudates are . collected and centrifuged at 6000xG for 15 minutes.
The rats's stomachs are removed and the gastric mucosa is detached from the underlying layers and by means of a 10 mm diameter die, mucosa samples from an area between the gastric corpus and pyloric antrum are taken. The mucosal PGE2 is extracted. PGE2 concentrations from the inflammatory exudates and the gastric mucosa are determined by means of inmmunoassay reagents of Cayman Chemical kit, Ann Arbor, Michigan, U.S.A. (Catalogue number: 514010) according to the manufacturer's instructions.

The present invention is illustrated below with the aid of examples. These illustrations are given solely by way of example and do not limit the the present invention.

### Examples:

### Example 1:

### Synthesis of (3,5-di-tert-butyl-4-hydroxy-phenyl)-(3-hydroxy-azetidin-1-yl)-methanone

### a) 3,5-Di-tert-butyl-4-hydroxy-benzoyl chloride

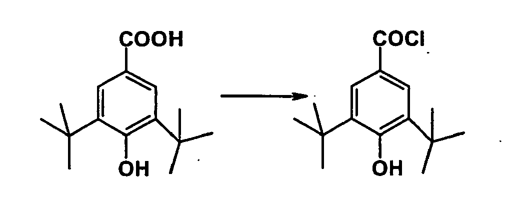

3,5-Di-tert-butyl-4-hydroxy-benzoic acid (16,6 g, 66,3 mmoles) was dissolved in chloroform (150 ml) and Thionyl chloride (10 ml, ≈139 mmoles) was added. The resulting mixture was refluxed for 9 hours, cooled to room temperature (approximately 25°C) and evaporated to dryness under reduced pressure. 17,6 g (99% of theoretical yield) of 3,5-di-tert-butyl-4-hydroxy-benzoyl chloride were obtained in form of yellow solid, which was used in the following reaction step without purification.
IR (KBr, cm⁻¹): 3555, 295.8, 1736, 1125.

### b) (3,5-Di-tert-butyl-4-hydroxy-phenyl)-(3-hydroxy-azetidin-1-yl)-methanone

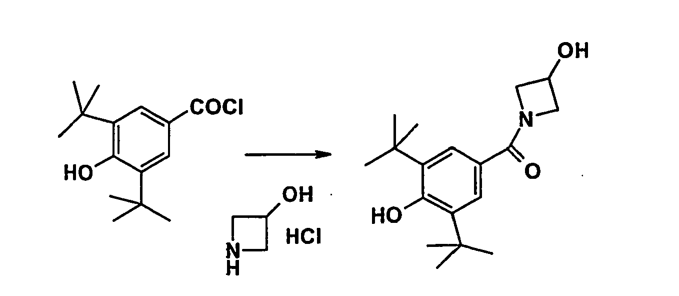

Azetidin-3-ol hydrochloride (2g, 18,3 mmoles) was dissolved in a 5 % weight/weight (45 ml) of an aqueous solution of sodium hydroxide, the resulting solution cooled to-5 °C and under vigorous stirring 3,5-di-tert-butyl-4-hydroxy-benzoyl chloride (5,4 g, 20 mmoles) obtained according to step (a) dissolved in 8 ml of THF was added. Afterwards the cooling bath was removed, the reaction mixture warmed to room temperature (approximately 25 °C) and stirred for an additional hour under these conditions. The reaction mixture was then extracted several times with diethyl ether, the etherical phases combined, washed with water, dried with sodium sulfate and evaporated to dryness to obtain 1,9 g of the crude product, which is crystallized from ethyl acetate-petroleum ether. 1,6 g (30% of theoretical yield) of (3,5-di-tert-butyl-4-hydroxy-phenyl)-(3-hydroxy-azetidin-1-yl)-methanone were obtained in crystalline form.
Melting point = 185-189°C
IR (KBr, cm⁻¹): 3506, 3262, 2956, 1611, 1572, 1449, 1421, 1406, 1113.
¹H-NMR (CDCl₃, δ): 1,43 (s, 18H), 2,8 (bs, 1 H), 4,0-4,2 (m, 2H), 4,45 (m, 2H), 4,7 (m, 1 H), 5,5 (s, 1 H), 7,5 (s, 2H).

### Example 3: .

### (3,5-di-tert-butyl-4-hydroxy-phenyl)-(3-hydroxy-3-methyl-azetidin-I -yl)-methanone.

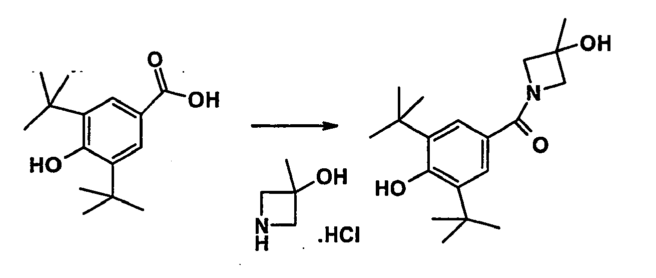

3-Methyl-azetidin-3-ol hydrochloride (0,6 g, 4,84 mmoles) was suspended in tetrahydrofuran (50 ml) and Triethylamine (1,2 ml) was added. The mixture was stirred at room temperature (approximately 25 °C) for 30 minutes and 3,5-di-tert-butyl-4-hydroxy benzoic acid (1,26 g, 5,1 mmoles) was added in one portion, the mixture was cooled to 0 °C and subsequently a solution of dicyclohexylcarbodiimide (1 g, 4,84 mmoles) in tetrahydrofuran (27 ml) was added. The reaction mixture was then heated to reflux for 3,5 hours, cooled and the insoluble solid was filtered off. The filtrate was concentrated using a rotavapor, the remaining solid dissolved in ethyl acetate, washed with water, dried over magnesium sulfate, filtered and evaporated to dryness. The crude residue obtained is crystallized from diethyl ether to give 1,14 g (73% of theoretical yield) of (3,5-di-tert-butyl-4-hydroxy-phenyl)-(3-hydroxy-3-methyl-azetidin-1-yl)-methanone.
Melting point = 148-153°C.
IR (KBr, cm⁻¹): 3497, 3274, 2963, 1612, 1598, 1415, 1235, 1120.
¹H-NMR (CDCl₃, δ): 1,4 (s, 18H), 1,5 (s, 3H), 3,9 (s, 1H), 4,1 (m, 3H), 4,3 (m, 1H), 5,5 (s, 1 H), 7,5 (s, 2H).

### Example 15:

### Synthesis of [1-(3,5-di-tert-butyl-4-hydroxy-benzyl)-azetidin-3-yl]-N-hydroxy-urea

### (a) [1-(3,5-Di-tert-butyl-4-hydroxy-benzyl)-azetidin-3-yl]-N-hydroxy-carbamic acid phenyl ester

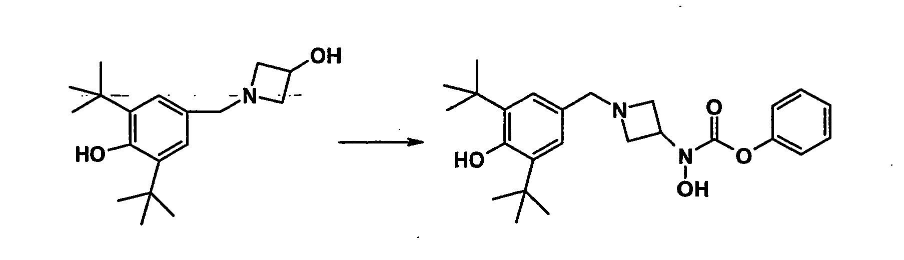

1-(3,5-di-tert-butyl-4-hydroxy-benzyl)-azetidin-3-ol (2.,27 g, 7,8 mmols), N,O bis-(phenoxycarbonyl)hydroxylamino (2,36 g, 8,58 mmols) (prepared according to the method described in A.O. Stewart and D.W. Brooks, J. Org. Chem., 57(18), 1992, 5020-5023. The respective part of the description is introduced by reference and forms part of the disclosure), and Triphenylphosphine (2,45 g, 9.36 mmols) were dissolved in 80 ml of anhydrous Tetrahydrofurane. The reaction mixture was cooled to 0°C under a nitrogen atmosphere, a solution of diisopropylazodicarboxylate (1,84 ml, 9.36 mmols) was added dropwise and the mixture was stirred at 0°C for one hour. Afterwards the mixture was allowed to warm up to room temperature and stirred overnight. The solvent was removed under reduced pressure via a rotavapor and the crude product was purified via column chromatography (silica gel, eluent: CHCl₃). After crystallization from diethyl ether 1,53 g (46% of theoretical yield) of the desired product were obtained as a white solid having a melting point of 161-163 °C.
IR (KBr, cm⁻¹): 3540, 3420, 2958, 1720, 1440, 1198, 760
¹H-NMR (CDCl₃, δ): 1,4 (s, 18H), 3,5 (t, 2H), 3,6 (m+s, 4H), 4,75 (m, 1H), 5,2 (bs, 1H), 7,05 (m, 3H), 7,25 (d, 1 H), 7,3 (m, 3H).

### (b) [1-(3,5-di-tert-butyl-4-hydroxy-benzyl)-azetidin-3-yl]-N-hydroxy-urea

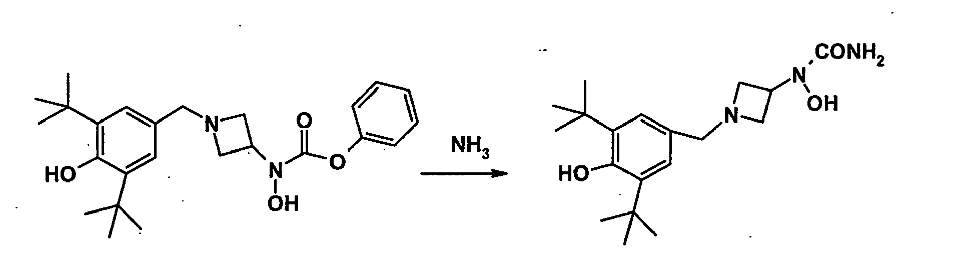

The product obtained according to step (a) (1,53 g, 3,6 mmols) was dissolved in 50 ml of Methanol and the solution was cooled to -78°C under nitrogen atmosphere. A solution of 5.4 ml of NH₃ condensed in 12.7 ml of Methanol at -78°C was added, the mixture was allowed to warm up to room temperature and stirred at this temperature in a closed reactor, thereby controlling the progress of the reaction via Thin-layer-chromatography (TLC) after two hours. If any unreacted starting product is detected another portion of NH₃ may be added and the reaction kept stirring overnight. The solution was concentrated using a rotavapor and the crude material was purified via column chromatography (silica gel, eluent: cloroform/methanol 95:5 (volume/volume) to give 0,58 g of the desired product (47% of theoretical yield) having a melting point of 90-95°C.
IR (KBr, cm⁻¹) :3650, 3494, 3338, 2903, 1656, 1569, 1431, 1363, 1213.
¹H-NMR (CDCl₃, δ): 1,4 (s, 18H), 3,5 (m, 4H), 3,6 (s, 2H), 4,8 (m, 1H), 5,2 (bs, 1H), 5,6 (bs, 2H), 7,0 (s, 2H).

### Example 16:

### Synthesis of (2S,3R)-N-[1-(3,5-di-tert-butyl-4-hydroxy-benzoyl)-2-methyl-azetidin-3-yl]-2,2,2-trifluoro-acetamide

### a) 3,5-Di-tert-butyl-4-hydroxy-benzoyl chloride

3,5-Di-tert-butyl-4-hydroxy-benzoyl chloride was prepared according to step (a) of example 1.

### b) N-[1-(3,5-di-tert-butyl-4-hydroxy-benzoyl)- (2S,3R)-2-methyl-azetidin-3-yl]-2,2,2-trifluoro-acetamide

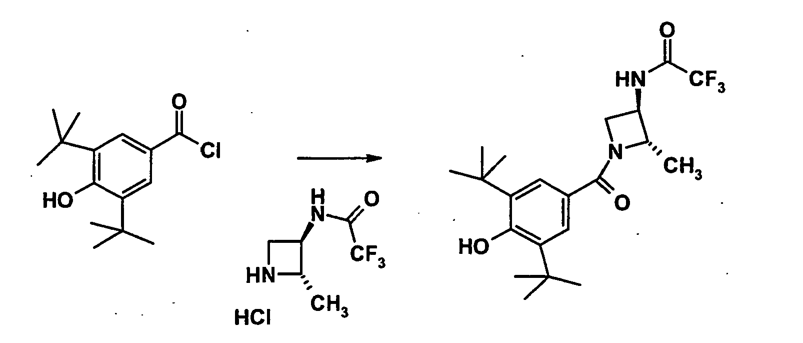

(2S,3R)-2,2,2-trifluoro-N-(2-methyl-azetidin-3-yl)-acetamide (1,04 g, 4,74 mmoles) hydrochloride was dissolved in dichloromethane (30 ml) and triethylamine (2,7 ml) was added, the mixture stirred for 10 minutes at room temperature, cooled to 0°C and a solution of 3,5-di-tert-butyl-4-hydroxy-benzoyl chloride (1,16 g, 4,3 mmoles) in dichloromethane (20 ml) was added. The reaction mixture was stirred at room temperature overnight, then poured on ice, the phases were separated, the aqeous phase extracted with dichloromethane and the combined organic phases were dried over magnesium sulfate, filtered and evaporated to dryness to give 1,42 g (80% of theoretical yield) of N-[1-(3,5-di-tert-butyl-4-hydroxy-benzoyl)-(2S,3R)-2-methyl-azetidin-3-yl]-2,2,2-trifluoro-acetamide.
IR (KBr, cm⁻¹) 2967, 1722, 1618, 1560, 1420, 1225, 1187, 1160.
¹H NMR (CDCl₃, δ): 1,4 (s, 18H), 1,5 (s, 3H), 4,3 (m, 2H), 4,7 (m, 2H), 5,5 (s, 1H), 7,3 (s, 2H), 9,0 (d, 1 H)

### Example 17:

### Synthesis of 1-(3,5-di-tert-butyl-4-hydroxy-benzyl)-azetidin-3-ol

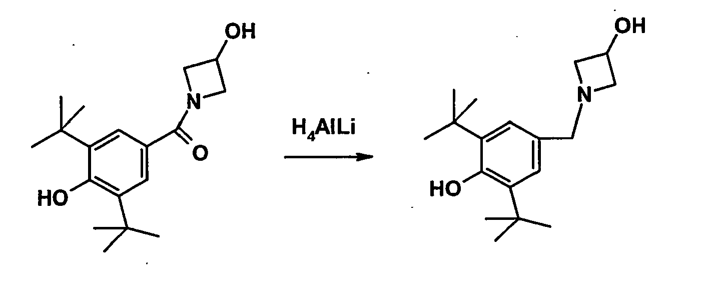

A suspension of lithium aluminium hydride (1,35 g, 36,7 mmoles) in anhydrous tetrahydrofuran (60 ml) was cooled to 0°C, and a solution of (3,5-di-tert-butyl-4-hydroxy-phenyl)-(3-hydroxy-azetidin-1-yl)-methanone (1,78 g, 6,12 mmoles) in anhydrous tetrahydrofuran (40 ml) was added. The cooling bath was removed, the mixture was stirred at room temperature overnight and then heated to reflux for 5 hours. The mixture was then cooled to 0°C and the excess of the reducing agent was eliminated by the addition of a saturated solution of ammonium chloride. The mixture is filtered and the filtrate was evaporated to dryness by use of a rotavapor. The residue was dissolved in diethyl ether, the resulting solution washed with water, dried over sodium sulfate and evaporated to dryness with a rotavapor. The resulting crude solid was crystallized from chloroform/petrol ether to give 1,33 g (75% of theoretical yield) of the desired product having a melting point of 139-142°C.
IR (KBr, cm⁻¹): 3513, 3331, 3076, 2956, 1434, 1360, 1162, 788.
¹H NMR (CDCl₃, δ) : 1,4 (s, 18H), 2,7 (bs, 1H), 2,9 (dd, 2H), 3,5 (s, 2H), 3,6 (dd, 2H), 4,4 (m, 1H), 5,1 (s, 1 H), 7,0 (s, 2H).

### Example 19:

### Synthesis of 3-hydroxy-azetidine-1-carboxylic acid 3,5-di-tert-butyl-phenyl ester

### (a) 3,5-Di-tert-butyl-phenyl chloroformiate

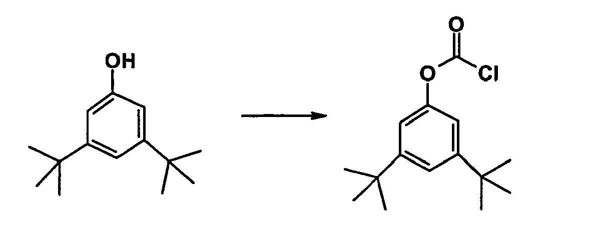

A solution of 3,5-di-tert-butyl-phenol (3,6 g, 17,4 mmoles) in 30 ml of tetrahydrofuran was cooled to 0°C and a solution of trichloro methyl chloroformiate (2,9 ml, 22,56 mmoles) in 20 ml of tetrahydrofurane was added. The cooling bath was removed and the mixture was stirred at room temperature overnight. The solution so obtained was used in the following step.

### (b) 3-Hydroxy-azetidine-1-carboxylic acid 3,5-di-tert-butyl-phenyl ester

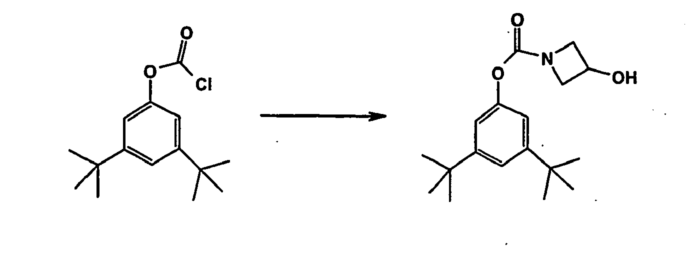

3-azetidinol hydrochloride (2,47 g, 22,62 mmoles) was suspended in tetrahydrofurane (30 ml), triethylamine (19 ml) was added and the mixture was heated to reflux for 30 minutes. The mixture was then cooled to room temperature and the solution of the chloroformiate according to step (a) was slowly added via a canule. The reaction mixture was heated to reflux overnight, cooled and filtered. The filtrate is evaporated to dryness in a rotavapor and the residue is purified via column chromatography (silica gel, eluent: ethyl acetate petrol ether 3:7 volume/volume). 0,83 g of 3-hydroxy-azetidine-1-carboxylic acid 3,5-di-tert-butyl-phenyl ester were obtained in form of a white solid having a melting point of 166-70°C.
IR (KBr, cm⁻¹): 3481, 2962, 1700, 1612, 1400.
¹H NMR (CDCl₃, δ) : 1,3 (s, 18H), 2,7 (m, 1H), 4,0 (m, 2H), 4,3 (m, 2H), 4,6 (m, 1H), 6,9 (s, 2H), 7,3 (s, 1 H).

The compounds of examples 2 and 11 were prepared according to the method described in example 1. The compounds of examples 4, 7, 9, 10, 12, 13, 17 and 18 were prepared according to the method described in example 3. The compound of example 14 was prepared analoguesly to the method described in J.M. Janusz et al., J. Med. Chem., 1998, 41 (7), 1112-1123. The respective description is incorporated by reference and forms part of the disclosure.

The compounds of examples 1-4, 7, and 9-19 and their spectroscopic data is given in the following tables 1 and 2:

**Table 1:**

| | | | | |
|---|---|---|---|---|
| 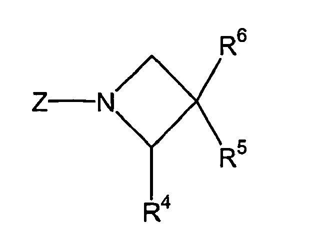 | | | | |

| **Z** | **Example** | **R⁴** | **R⁵** | **R⁶** |
|---|---|---|---|---|
| 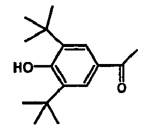 | 1 | H | OH | H |
| 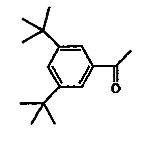 | 2 | H | OH | H |
| 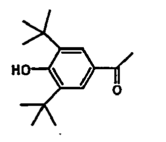 | 3 | H | OH | CH₃ |
| 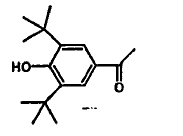 | 4 | CH₃ | OH | H |
| 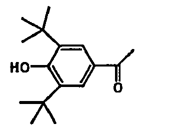 | 7 | H | Br | H |
| 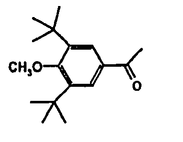 | 9 | H | OH | H |
| 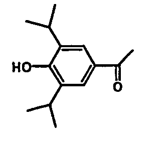 | 10 | H | OH | H |
| 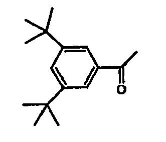 | 11 | H | O(CH₂)₄ONO₂ | H |
| 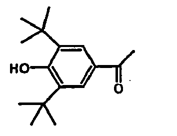 | 12 | Ph | OH | H |
| 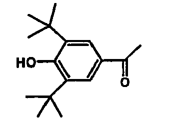 | 13 | H | Ph | OH |
| 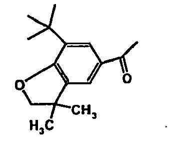 | 14 | H | OH | H |
| 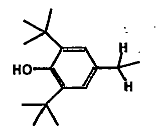 | 15 | H | N(OH)CONH₂ | H |
| 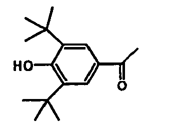 | 16 | CH₃ | NHCOCF₃ | H |
| 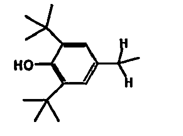 | 17 | H | OH | H |
| 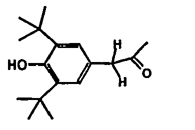 | 18 | H | OH | H |
| 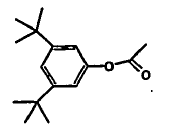 | 19 | H | OH | H |

**Table 2:**

| Example | Melting point °C | IR (KBr, cm⁻¹) | ¹H NMR (CDCl₃,δ) |
|---|---|---|---|
| 1 | 185-9 | 3506, 3262, 2956, 1611, 1572, 1449, 1421, 1406, 1113 | 1,43 (s, 18H), 2,8 (bs, 1 H), 4,0-4,2 (m, 2H), 4,45 (m, 2H), 4,7 (m, 1H), 5,5 (s, 1H), 7,5 (s, 2H) |
| 2 | 146-9 | 3444,3344,3287,2957, 1613, 1587, 1456, 1125 | 1,3 (s, 18H), 3,5 (d, 1H), 4,0-4,2 (m, 2H), 4,4 (dd, 2H), 4,6 (m, 1 H), 7,4 (s, 2H), 7,5 (s, 1 H). |
| 3 | 148-53 | 3497, 3274, 2963, 1612, 1598, 1415, 1235, 1120 | 1,4 (s, 18H), 1,5 (s, 3H), 3,9 (s, 1H), 4,1 (m, 3H), 4,3 (m, 1 H), 5,5 (s, 1 H), 7,5 (s, 2H). |
| 4 | 162-6 | 3469, 3250, 2960, 1608, 1571, 14011239, 1090 | 1,4 (s, 21 H), 3,3 (bs, 1 H), 3,9 (m, 1H), 4,1 (m, 1H), 4,4 (m, 2H), 5,5 (s, 1H), 7,5 (s, 2H). |
| 7 | 160-3 | 3506, 2953, 1632, 1600, 1383,-1131, 1110 | 1,4 (s, 18H), 4,4-4,8 (m, 5H), 7,5 (s, 2H) |
| 9 | 115-120 | 3296, 2963, 1602, 1555, 1474,1449,1410,1396 | 1,4 (s, 18H), 3,7 (s, 3H), 3,9-4,2 (m, 3H), 4,4 (m, 2H), 4,7 (m, 1H), 7,5 (s, 2H) |
| 10 | 156-60 | 3330, 1615, 1605, 1565, 1463, 1433, 1412, 1202 | 1,3 (s, 12H), 3,4 (m, 2H), 4,1-4,3 (m, 3H), 4,4-4,6 (m+s, 3H), 4,7 (m, 1H), 7,4 (s, 2H) |
| 11 | oil | 2962, 1637, 1594, 1459, | 1,3 (s, 18H), 1,7 (m, 2H), 1,8 (m, 2H), 3,4 (m, 2H), 4,0-4,15 (m, 2H), 4,3-4,4 (m, 3H), 4,5 (t, J=6,3Hz, 2H), 7,4 (s, 2H), 7,5 (s, 1H) |
| 12 | 178-81 | 3400, 3310, 2956, 1607, 1570, 1395, | 1,3 (s, 18H), 3,7 (d, 1H), 4,1 (dd, 1H), 4,3 (m, 1H), 4,6 (m, 1H), 5,3 (bs, 1H), 5,45 (bs, 1H), 7,3-7,4 (m, 7H) |
| 13 | 163-5 | 3530-3300, 2956, 1606, 1452, 1116 | 1,43 (s, 18H), 3,3 (bs, 1H), 4,4-4,6 (m, 4H), 5,5 (s, 1H), 7,4 (m, 3H), 7,5 (m, 4H) |
| 14 | 144-8 | 3265, 2955, 1603, 1582, 1450,1410,1129,995, 956 | 1,31 (s, 6H), 1,34 (s, 9H), 3,2 (bs, 1H), 4,0-4,3 (m+s, 4H), 4,45 (m, 2H), 4,7 (m, 1 H), 7,3 (s, 1 H), 7,35 (s, 1 H). |
| 15 | 90-5 | 3650, 3494, 3338, 2903, 1656, 1569, 1431, 1363, 1213 | 1,4 (s, 18H), 3,5 (m, 4H), 3,6 (s, 2H), 4,8 (m, 1H), 5,2 (bs, 1H), 5,6 (bs, 2H), 7,0 (s, 2H) |
| 16 | amorph | 2967, 1722, 1618, 1560, 1420, 1225, 1187, 1160 | 1,4 (s, 18H), 1,5 (s, 3H), 4,3 (m, 2H), 4,7 (m, 2H), 5,5 (s, 1H), 7,3 (s, 2H), 9,0 (d, 1H) |
| 17 | 139-42 | 3513, 3331, 3076, 2956, 1434, 1360, 1162, 788 | 1,4 (s, 18H), 2,7 (bs, 1H), 2,9 (dd, 2H), 3,5 (s, 2H), 3,6 (dd, 2H), 4,4 (m, 1H), 5,1 (s, 1H), 7,0 (s, 2H) |
| 18 | 154-6 | 2295, 2955, 1637, 1435, 1100 | 1,43 (s, 18H), 3,0 (d, 1H), 3,36 (s, 2H), 3,8 (dd, 1H), 4,0 (dd, 1H), 4,2 (t, 1H), 4,3 (t, 1H), 4,6 (m, 1H), 7,0 (s, 2H) |
| 19 | 166-70 | 3481, 2962, 1700, 1612, 1400 | 1,3 (s, 18H), 2,7 (m, 1H), 4,0 (m, 2H), 4,3 (m, 2H), 4,6 (m, 1H), 6,9 (s, 2H), 7,3 (s, 1 H) |

### Pharmacological Data:

### I. Cox-1/Cox-2 enzyme assay:

The Cox-1 /Cox-2 enzyme assay for the inventive azetidine compounds was carried out as described above. The values for enzyme inhibition of some inventive compounds are given in the following table I.

**Table I:**

| Compound according to Example | % inhibition 5 x 10⁻⁵M | | IC₅₀ µM | |
|---|---|---|---|---|
| | COX-1 | COX-2 | COX-1 | COX-2 |
| 1 | -- | -- | 37,7 | 1,51 |
| 2 | 9 | 78 | -- | 1,8 |
| 3 | -- | -- | 349 | 3,51 |
| 4 | -- | -- | 246 | 3,75 |
| 7 | 35 | -- | -- | 0,2 |
| 14 | -- | -- | 48,3 | 0,6 |
| 18 | 4 | 1 | -- | -- |

### II. Determination of Cox-1- and Cox-2-activity in human whole blood

The Cox-1- and Cox-2-activity in human whole blood is determined as described above. The values of some inventive compounds are given in the following table II.

**Table II:**

| Compound according to Example | hWB inhibition COX-1 IC₅₀ (µM) | hWB inhibition COX-2 IC₅₀ (µM) |
|---|---|---|
| 1 | 0,1 | 0,2 |
| 2 | 0,5 | 0,8 |
| 3 | 0,1 | 0,1 |
| 4 | 0,5 | 0,5 |
| 14 | 0,05 | 0,1 |

### III: Analgesia Test in rats

The test of the inventive compounds for analgesic activity was carried out as described above. The values for some of the inventive compounds is given in the following table III.

**Table III:**

| Compound according to Example | ED₅₀ (mg/kg) |
|---|---|
| 1 | 0,4 |
| 2 | 1,65 |
| 3 | 0,14 |
| 4 | 0,7 |
| 14 | 3 |

### IV. Test for activity against Edema in rats

The test for activity against edema was carried out as described above. The values of some of the inventive compounds are given in the following table IV:

**Table IV:**

| Compound according to Example | ED₅₀ (mg/kg) |
|---|---|
| 1 | 3 |
| 2 | 28 |
| 3 | 58 |
| 4 | 31 |
| 17 | 62 |

### V: Test for antiarthritic activity in rats

The test for antiarthritic activity was carried out as described above. The values of some of the inventive compounds are given in the following table V.

**Table V:**

| **Compound according to Example** | ED₅₀ (mg/kg) |
|---|---|
| 1 | 0,5 |
| 3 | 0,34 |

### VI: PGE2 production in rat inflammatory exudate and gastric mucosa

The PGE2 production in rat inflammatory exudate and gastric mucosa was carried out as described above. The values for some of the inventive compounds are given in the following table VI.

**Table VI:**

| Example | PGE₂ gastric mucose ED₅₀ (mg/kg) | PGE₂ Inflam. exudade ED₅₀ (mg/kg) |
|---|---|---|
| 1 | 0,16 | 0,28 |
| 2 | 3,7 | 5,9 |
| 3 | 7,3 | 0,5 |
| 4 | 1,0 | 5,8 |
| 14 | 2,7 | 1,8 |

## Claims

1. Substituted Azetidine compounds of general formula I, 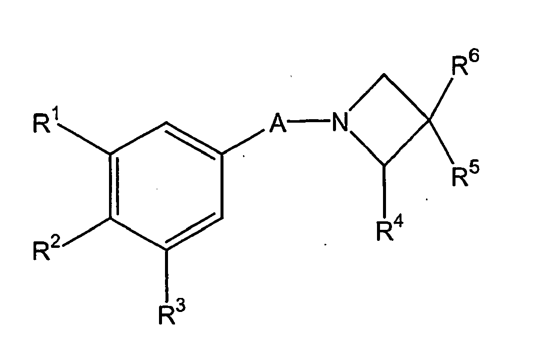 wherein
A represents a -C=O-moiety, a -CH₂-moiety, a -CH₂-C=O-moiety bonded to the azetidine ring via its carbonyl carbon atom, or a -O-C(=O)-moiety bonded to the azetidine ring via its carbonyl carbon atom,
R¹, R³, identical or different, represent a hydrogen atom or a linear or branched, saturated or unsaturated C₁₋₄-aliphatic group,
R² represents a hydrogen atom, a hydroxyl group or a C₁₋₃-alkoxy group,
or R¹ and R² or R² and R³ together form an -O-CH₂-CH₂-chain, which is optionally substituted with one or more methyl groups
with the proviso that R¹, R² and R³ do not identically represent a hydrogen atom, and if A represents a -CH₂-moiety, then at least two of the residues R¹, R² and R³ do not identically represent a hydrogen atom,
R⁴ represents a hydrogen atom, an optionally at least mono-substituted aryl group, or a linear or branched, saturated or unsaturated aliphatic group, which may be substituted by one or more substituents independently selected from the group consisting of hydroxy, fluorine, chlorine, bromine, branched or unbranched C₁₋₄-alkoxy, branched or unbranched C₁₋₄-perfluoroalkoxy and branched or unbranched C₁₋₄-perfluoroalkyl,
R⁵ represents a hydrogen atom, a halogen atom, a hydroxyl group, a linear or branched, saturated or unsaturated, optionally at least mono-substituted aliphatic group, an -OR⁷-moiety, -an -NH₂-moiety, a -CO-NH₂-moiety, an -NH-CO-R⁸-moiety, an -N(OH)-CO-NH₂-moiety, an -O(CH₂)₁₋₄-ONO₂-moiety, an optionally at least mono-substituted aryl group, or a carboxy-group,
R⁶ represents a hydrogen atom, a halogen atom, a hydroxyl group; a linear or branched, saturated or unsaturated, optionally at least mono-substituted aliphatic group, an -OR⁹-moiety, -an -NH₂-moiety, a -CO-NH₂-moiety, an -NH-CO-R¹⁰-moiety, an -N(OH)-CO-NH₂-moiety, an optionally at least mono-substituted aryl group, or a carboxy-group,
R⁷, R⁸, R⁹, R¹⁰, independent from one another, represent a linear or branched, saturated or unsaturated, optionally at least mono-substituted aliphatic group,
with the provisos
that if A represents a -(C=O)-moiety, R⁴ represents a hydrogen atom and one of the residues R⁵ and R⁶ represents a hydrogen atom, then the other one of these residues R⁵ and R⁶ does not represent an -NH₂-moiety, a -CONH₂-moiety, or a methyl group, which is substituted by an -NH₂-moiety or an azaheterocycle, and
if A represents a -C=O-moiety, a -CH₂-C=O-moiety bonded to the azetidine ring via its carbonyl carbon atom, or a -O-C(=O)-moiety bonded to the azetidine ring via its carbonyl carbon atom and one of the residues R⁵ and R⁶ represents a hydrogen atom or an optionally at least mono-substituted, linear or branched, saturated or unsaturated aliphatic group, then the other one of these residues R⁵ and R⁶ does not represent an -NH₂- or a COOH-moiety,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

2. Compounds according to claim 1, **characterized in that** R¹ and R³, identical or different, represent a hydrogen atom or a linear or branched C₁₋₄-alkyl group.

3. Compounds according to claim 1 or 2, **characterized in that** R¹ and R³ are identical and represent a C₁₋₄-alkyl group, preferably a C₃₋₄ alkyl group, more preferably an iso-propyl group or a tert.-Butyl group.

4. Compounds according to one or more of claims 1-3, **characterized in that** R² represents a hydrogen atom, a hydroxyl group or a methoxy group.

5. Compounds according to one or more of claims 1-4, **characterized in that** R⁴ represents a hydrogen atom, an optionally at least mono-substituted phenyl group, or a linear or branched, saturated or unsaturated C₁₋₆ aliphatic group, whereby said aliphatic group may be substituted by one or more substituents independently selected from the group consisting of hydroxy, fluorine, chlorine, bromine, branched or unbranched C₁₋₄-alkoxy, branched or unbranched C₁₋₄-perfluoroalkoxy and branched or unbranched C₁₋₄-perfluoroalkyl, preferably a hydrogen atom, a methyl group or an unsubstituted phenyl group.

6. Compounds according to one or more of claims 1-5, **characterized in that** R⁵ represents a hydrogen atom, a halogen atom, a hydroxyl group, a linear or branched, saturated or unsaturated, optionally at least mono-substituted C₁₋₆ aliphatic group, an -NH₂-moiety, a -CO-NH₂-moiety, an -NH-CO-R⁸-moiety, an - N(OH)-CO-NH₂-moiety, an -O(CH₂)₄-ONO₂-moiety, an optionally at least mono-substituted phenyl group, or a carboxy-group, preferably a hydrogen atom, a bromine atom, a hydroxyl group, an -NH₂-moiety, a -CO-NH₂-moiety, an -NH-CO-R⁸-moiety, an -N(OH)-CO-NH₂-moiety, an -O(CH₂)₄-ONO₂-moiety, an unsubstituted phenyl group, or a carboxy-group.

7. Compounds according to one or more of claims 1-6, **characterized in that** R⁶ represents a hydrogen atom, a halogen atom, a hydroxyl group, a linear or branched, saturated or unsaturated, optionally at least mono-substituted C₁₋₆ aliphatic group, an -NH₂-moiety, a -CO-NH₂-moiety, an -NH-CO-R⁸-moiety, anN(OH)-CO-NH₂-moiety, an optionally at least mono-substituted phenyl group, or a carboxy-group, preferably a hydrogen atom, a hydroxyl group or a methyl group.

8. Compounds according to one or more of claims 1-7, **characterized in that** R⁷, R⁸, R⁹, R¹⁰, independent from one another, represent a linear or branched, saturated or unsaturated, optionally at least mono-substituted C₁₋₆ aliphatic group, preferably a linear or branched, optionally at least mono-substituted C₁₋₆ alkyl group, more preferably a methyl group or an ethyl group, which is optionally perfluorinated.

9. Compounds according to one ore more of claims 1-8 of general formula 1, 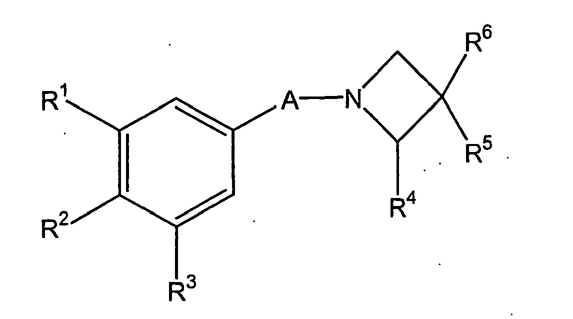 wherein
A represents a -C=O-moiety, a -CH₂-moiety, a -CH₂-C=O-moiety bonded to the azetidine ring via its carbonyl carbon atom, or a -O-C(=O)-moiety bonded to the azetidine ring via its carbonyl carbon atom,
R¹, R³ both identically represent a linear or branched C₁₋₄-alkyl group, preferably an iso-propyl group or a tert.-butyl group,
R² represents a hydrogen atom, a hydroxyl group or a linear or branched C₁₋₄-alkoxy group,
or R¹ and R² or R² and R³ together form an -O-CH₂-C(CH₃)₂-chain, whereby the oxygen atom of said chain is bonded to the 4-position of the phenyl ring,
R⁴ represents a hydrogen atom, a linear or branched C₁₋₄ alkyl group or an unsubstituted phenyl group,
R⁵ represents a fluorine atom, a chlorine atom, a bromine atom, a hydroxyl group, an -NH-CO-CF₃-moiety, an -N(OH)-CO-NH₂-moiety, an -O(CH₂)₄ONO₂-moiety, or an unsubstituted phenyl group,
R⁶ represents a hydrogen atom, a linear or branched C₁₋₄-alkyl group, or a hydroxyl group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

10. Compounds according to one ore more of claims 1-9 of general formula 1, 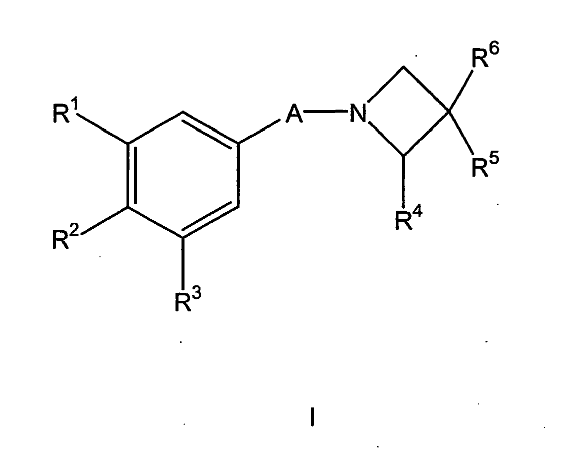 wherein
A represents a -C=O-moiety, a -CH₂-moiety, a -CH₂-C=O-moiety bonded to the azetidine ring via its carbonyl carbon atom, or a -O-C(=O)-moiety bonded to the azetidine ring via its carbonyl carbon atom,
R¹, R³ both identically represent an iso-propyl group or a tert.-butyl group,
R² represents a hydrogen atom, a hydroxyl group or a methoxy group,
or R¹ and R² or R² and R³ together form an -O-CH₂-C(CH₃)₂-chain, whereby the oxygen atom of said chain is bonded to the 4-position of the phenyl ring,
R⁴ represents a hydrogen atom, a methyl group or an unsubstituted phenyl group,
R⁵ represents a bromine atom, a hydroxyl group, -an -NH₂-moiety, a -CO-NH₂-moiety, an -NH-CO-CF₃-moiety, an -N(OH)-CO-NH₂-moiety, an -O(CH₂)₄ONO₂-moiety, an unsubstituted phenyl group, or a carboxy-group,
R⁶ represent a hydrogen atom, a methyl group or a hydroxyl group,
optionally in form of one of the stereoisomers, preferably enantiomers or diastereomers, a racemate or in form of a mixture of at least two of the stereoisomers, preferably enantiomers and/or diastereomers, in any mixing ratio, or a corresponding salt thereof, or a corresponding solvate thereof.

11. Compounds according to one or more of claims 1-10 selected from the group consisting of .
[1] (3,5-di-tert-butyl-4-hydroxy-phenyl)-(3-hydroxy-azetidin-1-yl)-methanone;
[2] (3,5-di-tert-butyl-phenyl)-(3-hydroxy-azetidin-1-yl)-methanone;
[3] (3,5-di-tert-butyl-4-hydroxy-phenyl)-(3-hydroxy-3-methyl-azetidin-1-yl)-methanone;
[4] (3,5-di-tert-butyl-4-hydroxy-phenyl)-(3-hydroxy-2-methyl-azetidin-1-yl)-methanone;
[7] (3-Bromo-azetidin-1-yl)-(3,5-di-tert-butyl-4-hydroxy-phenyl)-methanone;
[9] (3,5-di-tert-butyl-4-methoxy-phenyl)-(3-hydroxy-azetidin-1-yl)-methanone;
[10] (3-hydroxy-azetidin-1-yl)-(4-hydroxy-3,5-diisopropyl-phenyl)-methanone;
[11] (3,5-di-tert-butyl-phenyl)-[3-(4-nitrooxy-butoxy)-azetidin-1-yl]-methanone;
[12] (3,5-di-tert-butyl-4-hydroxy-phenyl)-(3-hydroxy-2-phenyl-azetidin-1-yl)-methanone;
[13] (3,5-di-tert-butyl-4-hydroxy-phenyl)-(3-hydroxy-3-phenyl-azetidin-1-yl)-methanone;
[14] (7-tert-butyl-3,3-dimethyl-2,3-dihydro-benzofuran-5-yl)-(3-hydroxy-azetidin-1-yl)-methanone;
[15] [1-(3,5-di-tert-butyl-4-hydroxy-benzyl)-azetidin-3-yl]-N-hydroxy-urea;
[16] N-[1-(3,5-di-tert-butyl-4-hydroxy-benzoyl)- (2S,3R)-2-methyl-azetidin-3-yl]-2,2,2-trifluoro-acetamide;
[17] 1-(3,5-di-tert-butyl-4-hydroxy-benzyl)-azetidin-3-ol;
[18] 2-(3,5-di-tert-butyl-4-hydroxy-phenyl)-1-(3-hydroxy-azetidin-1-yl)-ethanone;
[19] (3-hydroxy-azetidine-1-carboxylic acid)-3,5-di-tert-butyl-phenyl ester
optionally in form of a corresponding salt or a corresponding solvate.

12. Process for the preparation of substituted azetidine compounds of general formula I according to one or more of claims 1-11, **characterized in that** at least one compound of general formula II, 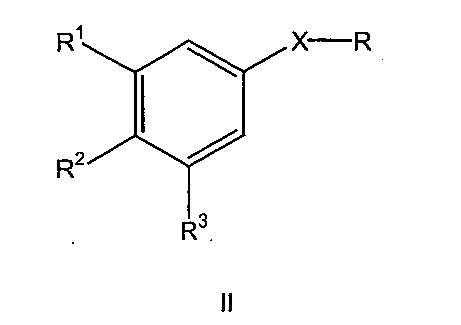 wherein R¹-R³ have the meaning according to one or more of claims 1-11, X represents a bond or an -(CH₂)-moiety and R represents a carboxy group or an activated carbonyl group, is reacted with at least one compound of general formula III, 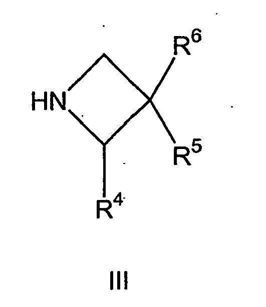 optionally in the form of a corresponding salt, wherein R⁴-R⁶ have the meaning according to one or more of claims 1-11, to yield a compound of general formula I according to one or more of claims 1-11, wherein A represents a -(C=O)-moiety or an -(CH₂)-CO-moiety, which is optionally purified and/or optionally isolated,
and optionally at least one compound of general formula I according to one or more of claims 1-11, wherein A represents a -(C=O)-moiety is reduced to yield at least one compound of general formula I according to one or more of claims 1-11, wherein A represents a -(CH₂)-moiety, which is optionally purified and/or isolated,
or at least one compound of general formula IV, 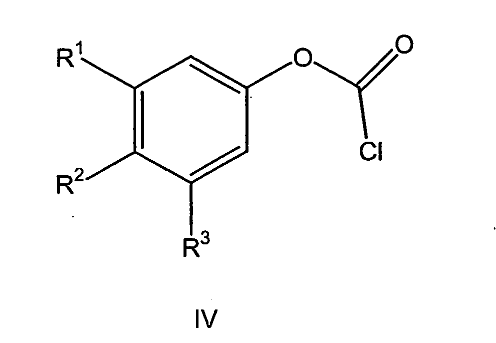 wherein R¹-R³ have the meaning according to one or more of claims 1-11, is reacted with at least one compound of general formula III given above, to yield at least one compound of general formula I according to one or more of claims 1-10, wherein A represents an O-(C=O)-moiety, and said compound is optionally purified and/or optionally isolated.

13. Medicament comprising at least one substituted azetidine compound according to one or more of claims 1-11 and optionally one or more pharmaceutically acceptable excipients.

14. Medicament according to claim 13 for the inhibition of Cyclooxygenase-1, for the prophylaxis and/or treatment of Cyclooxygenase-1 related disorders, for the inhibition of Cyclooxgenase-2 and/or for the prophylaxis and/or treatment of Cyclooxygenase-2 related disorders.

15. Medicament according to claim 13 or 14 for the prophylaxis and/or treatment of pain, for the prophylaxis and/or treatment of inflammation and/or for the prophylaxis and/or treatment of inflammation related disorders, whereby said inflammation-related disorders may preferably be selected from the group consisting of arthritis, rheumatoid arthritis, spondyloarthropathies, gouty arthritis, osteoarthritis, systemic lupus erythematosus, juvenile arthritis, rheumatic fever, symptoms associated with influenza or other viral infections, common cold, lower back pain, neck pain, dysmenorrhea, headache, toothache, sprains, strains, myositis, neuralgia, synovitis, gout, ankylosing spondylitis, bursitis, edema, inflammations following dental procedures, inflammations following dental procedures, vascular diseases, migraine headaches, periarteritis nodosa, thyroiditis, aplastic anemia, Hodkin's disease, sclerodoma, type I diabetes, myasthenia gravis, sarcoidosis, nephrotic syndrome, Behcet's syndrome, polymyositis, gingivitis, hypersensivity, conjunctivitis, swelling ocurring after injury and myocardia ischemia, for the prophylaxis and/or treatment of asthma, for the prophylaxis and/or treatment of bronchitis, for the prophylaxis and/or treatment of tendinitis, for the prophylaxis and/or treatment of bursitis, for the prophylaxis and/or treatment of skin related conditions, whereby said skin related conditions may preferably be selected from the group consisting of psoriasis, eczema, burns and dermatitis, for the prophylaxis and/or treatment of gastrointestinal disorders, whereby said gastrointestinal disorders may preferably be selected from the group consisting of inflammatory bowel disease, Crohn's disease, gastritis, irritable bowel syndrome and ulcerative colitis, or for treatment of fever, or for the prophylaxis and/or treatment of cancer or a cancer-related disorders, whereby said cancer or related disorder may preferably be selected from the group consisting of brain cancer, bone cancer, epithelial cell-derived neoplasia (epithelial carcinoma), basal cell carcinoma, adenocarcinoma, gastrointestinal cancer, lip cancer, colon cancer, liver cancer, bladder cancer, pancreas cancer, ovary cancer, cervical cancer, lung cancer, breast cancer, skin cancer, squamous cell cancer, prostate cancer, renal cell carcinoma and other known cancers that effect epithelial cells throughout the body, for the prophylaxis and/or treatment of polyps, for the prophylaxis and/or treatment of angiogenesis mediated disorders, preferably selected from the group consisting of metastasis, corneal graft rejection, ocular neovascularization, retinal neovascularisation, diabethic retinopathy, retrolental fibroplasia, neovascular glaucoma, gastric ulcer, infantile hemaginomas, angiofibroma of the nasopharynx, avascular necrosis of the bone and endometriosis.

16. Medicament according to one or more of claims 13-15 for the prophylaxis and/or treatment of pain.

17. Medicament according to one or more of claims 13-15 for the prophylaxis and/or treatment of inflammation.

18. Medicament according to one or more of claims 13-15 for the prophylaxis and/or treatment of inflammation related disorders, whereby said inflammation-related disorders may preferably be selected from the group consisting of arthritis, rheumatoid arthritis, spondyloarthropathies, gouty arthritis, osteoarthritis, systemic lupus erythematosus, juvenile arthritis, rheumatic fever, symptoms associated with influenza or other viral infections, common cold, lower back pain, neck pain, dysmenorrhea, headache, toothache, sprains, strains, myositis, neuralgia, synovitis, gout, ankylosing spondylitis, bursitis, edema, inflammations following dental procedures, inflammations following dental procedures, vascular diseases, migraine headaches, periarteritis nodosa, thyroiditis, aplastic anemia, Hodkin's disease, sclerodoma, type I diabetes, myasthenia gravis, sarcoidosis, nephrotic syndrome, Behcet's syndrome, polymyositis, gingivitis, hypersensivity, conjunctivitis, swelling ocurring after injury and myocardia ischemia.

19. Use of at least one substituted azetidine compound according to one or more of claims 1-11 and optionally one or more pharmaceutically acceptable excipients, for the preparation of a medicament for the inhibition of Cyclooxygenase-1, for the prophylaxis and/or treatment of Cyclooxygenase-1 related disorders, for the inhibition of Cyclooxgenase-2 and/or for the prophylaxis and/or treatment of Cyclooxygenase-2 related disorders.

20. Use of at least one substituted azetidine compound according to one or more of claims 1-11 and optionally one or more pharmaceutically acceptable excipients for the preparation of a medicament for the prophylaxis and/or treatment of pain, for the prophylaxis and/or treatment of inflammation and/or for the prophylaxis and/or treatment of inflammation related disorders, whereby said inflammation-related disorders may preferably be selected from the group consisting of arthritis, rheumatoid arthritis, spondyloarthropathies, gouty arthritis, osteoarthritis, systemic lupus erythematosus, juvenile arthritis, rheumatic fever, symptoms associated with influenza or other viral infections, common cold, lower back pain, neck pain, dysmenorrhea, headache, toothache, sprains, strains, myositis, neuralgia, synovitis, gout, ankylosing spondylitis, bursitis, edema, inflammations following dental procedures, inflammations following dental procedures, vascular diseases, migraine headaches, periarteritis nodosa, thyroiditis, aplastic anemia, Hodkin's disease, sclerodoma, type I diabetes, myasthenia gravis, sarcoidosis, nephrotic syndrome, Behcet's syndrome, polymyositis, gingivitis, hypersensivity, conjunctivitis, swelling ocurring after injury and myocardia ischemia, for the prophylaxis and/or treatment of asthma, for the prophylaxis and/or treatment of bronchitis, for the prophylaxis and/or treatment of tendinitis, for the prophylaxis and/or treatment of bursitis, for the prophylaxis and/or treatment of skin related conditions, whereby said skin related conditions may preferably be selected from the group consisting of psoriasis, eczema, burns and dermatitis, for the prophylaxis and/or treatment of gastrointestinal disorders, whereby said gastrointestinal disorders may preferably be selected from the group consisting of inflammatory bowel disease, Crohn's disease, gastritis, irritable bowel syndrome and ulcerative colitis, or for treatment of fever, or for the prophylaxis and/or treatment of cancer or a cancer-related disorders, whereby said cancer or related disorder may preferably be selected from the group consisting of brain cancer, bone cancer, epithelial cell-derived neoplasia (epithelial carcinoma), basal cell carcinoma, adenocarcinoma, gastrointestinal cancer, lip cancer, colon cancer, liver cancer, bladder cancer, pancreas cancer, ovary cancer, cervical cancer, lung cancer, breast cancer, skin cancer, squamous cell cancer, prostate cancer, renal cell carcinoma and other known cancers that effect epithelial cells throughout the body, for the prophylaxis and/or treatment of polyps, for the prophylaxis and/or treatment of angiogenesis mediated disorders, preferably selected from the group consisting of metastasis, corneal graft rejection, ocular neovascularization, retinal neovascularisation, diabethic retinopathy, retrolental fibroplasia, neovascular glaucoma, gastric ulcer, infantile hemaginomas, angiofibroma of the nasopharynx, avascular necrosis of the bone and endometriosis.

21. Use of at least one substituted azetidine compound according to one or more of claims 1-11 and optionally one or more pharmaceutically acceptable excipients for the preparation of a medicament for the prophylaxis and/or treatment of pain.

22. Use of at least one substituted azetidine compound according to one or more of claims 1-11 and optionally one or more pharmaceutically acceptable excipients < > for the prophylaxis and/or treatment of inflammation.

23. Use of at least one substituted azetidine compound according to one or more of claims 1-11 and optionally one or more pharmaceutically acceptable excipients for the preparation of a medicament for the prophylaxis and/or treatment of inflammation related disorders, whereby said inflammation-related disorders may preferably be selected from the group consisting of arthritis, rheumatoid arthritis, spondyloarthropathies, gouty arthritis, osteoarthritis, systemic lupus erythematosus, juvenile arthritis, rheumatic fever, symptoms associated with influenza or other viral infections, common cold, lower back pain, neck pain, dysmenorrhea, headache, toothache, sprains, strains, myositis, neuralgia, synovitis, gout, ankylosing spondylitis, bursitis, edema, inflammations following dental procedures, inflammations following dental procedures, vascular diseases, migraine headaches, periarteritis nodosa, thyroiditis, aplastic anemia, Hodkin's disease, sclerodoma, type I diabetes, myasthenia gravis, sarcoidosis, nephrotic syndrome, Behcet's syndrome, polymyositis, gingivitis, hypersensivity, conjunctivitis, swelling ocurring after injury and myocardia ischemia.

## Patentansprüche

1. Substituierte Azetidin-Verbindungen der allgemeinen Formel 1 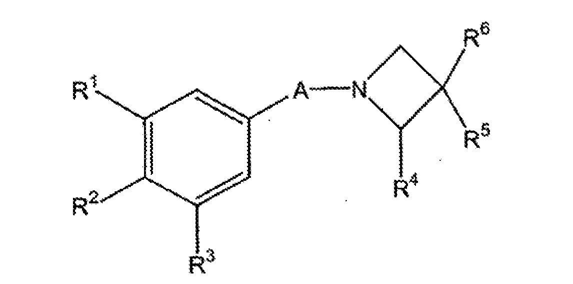 worin A eine -C=O-Einheit, eine -CH₂-Einheit, eine -CH₂-C=O-Einheit, die über ihr Carbonyl-Kohlenstoffatom an den Azetidin-Ring gebunden ist, oder eine -O-C(=O)-Einheit, die über ihr Carbonyl-Kohlenstoffatom an den Azetidin-Ring gebunden ist, repräsentiert,
R¹, R³ gleich oder verschieden sind und ein Wasserstoffatom oder eine lineare oder verzweigte, gesättigte oder ungesättigte C₁₋₄-Aliphatengruppe repräsentieren,
R² ein Wasserstoffatom, eine Hydroxylgruppe oder eine C₁₋₃-Alkoxygruppe repräsentiert,
oder R¹ und R² oder R² und R³ zusammen eine -O-CH₂-CH₂-Kette bilden, die gegebenenfalls mit einer oder mehreren Methylgruppen substituiert ist,
mit der Maßgabe, dass R¹, R² und R³ nicht identisch ein Wasserstoffatom repräsentieren, und wenn A eine -CH₂-Einheit repräsentiert, dass dann mindestens zwei der Reste R¹, R² und R³ nicht identisch ein Wasserstoffatom repräsentieren,
R⁴ ein Wasserstoffatom, eine gegebenenfalls mindestens mono-substituierte Arylgruppe oder eine lineare oder verzweigte, gesättigte oder ungesättigte Aliphatengruppe repräsentiert, die mit einem oder mehreren Substituenten substituiert sein kann, welche unabhängig aus der Gruppe ausgewählt sind, die aus Hydroxy, Fluor, Chlor, Brom, verzweigtem oder unverzweigtem C₁₋₄-Alkoxy, verzweigtem oder unverzweigtem C₁₋₄-Perfluoralkoxy und verzweigtem oder unverzweigtem C₁₋₄-Perfluoralkyl besteht,
R⁵ ein Wasserstoffatom, ein Halogenatom, eine Hydroxylgruppe, eine lineare oder verzweigte, gesättigte oder ungesättigte, gegebenenfalls mindestens mono-substituierte Aliphatengruppe, eine -OR⁷-Einheit, eine -NH₂-Einheit, eine -CO-NH₂-Einheit, eine - NH-CO-R⁸-Einheit, eine -N(OH)-CO-NH₂-Einheit, eine -O(CH₂)₁₋₄-ONO₂-Einheit, eine gegebenenfalls mindestens mono-substituierte Arylgruppe oder eine Carboxygruppe repräsentiert,
R⁶ ein Wasserstoffatom, ein Halogenatom, eine Hydroxylgruppe, eine lineare oder verzweigte, gesättigte oder ungesättigte, gegebenenfalls mindestens mono-substituierte Aliphatengruppe, eine -OR⁹-Einheit, eine -NH₂-Einheit, eine -CO-NH₂-Einheit, eine - NH-CO-R 10 -Einheit, eine -N(OH)-CO-NH₂-Einheit, eine gegebenenfalls mindestens mono-substituierte Arylgruppe oder eine Carboxygruppe repräsentiert,
R⁷, R⁸, R⁹, R¹⁰ unabhängig voneinander eine lineare oder verzweigte, gesättigte oder ungesättigte, gegebenenfalls mindestens mono-substituierte Aliphatengruppe repräsentieren,
mit der Maßgabe, dass
wenn A eine -(C=O)-Einheit repräsentiert, R⁴ ein Wasserstoffatom repräsentiert, und einer der Reste R⁵ und R⁶ ein Wasserstoffatom repräsentiert, dann der andere dieser Reste R⁵ und R⁶ keine -NH₂-Einheit, keine -CONH₂-Einheit oder keine Methylgruppe, die mit einer -NH₂-Einheit oder einem Azaheterocyclus substituiert ist, repräsentiert, und
wenn A eine -C=O-Einheit, eine -CH₂-C=O-Einheit, die über ihr Carbonyl-Kohlenstoffatom an den Azetidin-Ring gebunden ist, oder eine -O-C(=O)-Einheit, die über ihr Carbonyl-Kohlenstoffatom an den Azetidin-Ring gebunden ist, repräsentiert, und einer der Reste R⁵ und R⁶ ein Wasserstoffatom oder eine gegebenenfalls mindestens mono-substituierte, lineare oder verzweigte, gesättigte oder ungesättigte Aliphatengruppe repräsentiert, dass dann der andere dieser Reste R⁵ und R⁶ keine -NH₂- oder COOH-Einheit repräsentiert,
gegebenenfalls in Form eines der Stereoisomere, bevorzugt Enantiomere oder Diastereomere, eines Racemats oder in Form einer Mischung von mindestens zweien der Stereoisomere, bevorzugt Enantiomere und/oder Diastereomere, in jedem Mischungsverhältnis, oder ein korrespondierendes Salz davon oder ein korrespondierendes Solvat davon.

2. Verbindungen nach Anspruch 1, **dadurch gekennzeichnet, dass** R¹ und R³ gleich oder verschieden sind und ein Wasserstoffatom oder eine lineare oder verzweigte C₁₋₄-Alkylgruppe repräsentieren.

3. Verbindungen nach Anspruch 1 oder 2, **dadurch gekennzeichnet, dass** R¹ und R³ gleich sind und eine C₁₋₄-Alkylgruppe, bevorzugt eine C₃₋₄-Alkylgruppe, bevorzugter eine iso-Propylgruppe oder eine tert-Butylgruppe repräsentieren.

4. Verbindungen nach einem oder mehreren der Ansprüche 1 bis 3, **dadurch gekennzeichnet, dass** R² ein Wasserstoffatom, eine Hydroxylgruppe oder eine Methoxygruppe repräsentiert.

5. Verbindungen nach einem oder mehreren der Ansprüche 1 bis 4, **dadurch gekennzeichnet, dass** R⁴ ein Wasserstoffatom, eine gegebenenfalls mindestens mono-substituierte Phenylgruppe oder eine lineare oder verzweigte, gesättigte oder ungesättigte C₁₋₆-Aliphatengruppe repräsentiert, wobei die Aliphatengruppe mit einem oder mehreren Substituenten substituiert sein kann, welche unabhängig aus der Gruppe ausgewählt sind, die aus Hydroxy, Fluor, Chlor, Brom, verzweigtem oder unverzweigtem C₁₋₄-Alkoxy, verzweigtem oder unverzweigtem C₁₋₄-Perfluoralkoxy und verzweigtem oder unverzweigtem C₁₋₄-Perfluoralkyl, bevorzugt einem Wasserstoffatom, einer Methylgruppe oder einer unsubstituierten Phenylgruppe besteht.

6. Verbindungen nach einem oder mehreren der Ansprüche 1 bis 5, **dadurch gekennzeichnet, dass** R⁵ ein Wasserstoffatom, ein Halogenatom, eine Hydroxylgruppe, eine lineare oder verzweigte, gesättigte oder ungesättigte, gegebenenfalls mindestens mono-substituierte C₁₋₆-Aliphatengruppe, eine -NH₂-Einheit, eine -CO-NH₂-Einheit, eine -NH-CO-R⁸-Einheit, eine -N(OH)-CO-NH₂-Einheit, eine -O(CH₂)₄-ONO₂-Einheit, eine gegebenenfalls mindestens mono-substituierte Phenylgruppe oder eine Carboxygruppe, bevorzugt ein Wasserstoffatom, ein Bromatom, eine Hydroxylgruppe, eine -NH₂-Einheit, eine -CO-NH₂-Einheit, eine -NH-CO-R⁸-Einheit, eine -N(OH)-CO-NH₂-Einheit, eine -O(CH₂)₄-ONO₂-Einheit, eine unsubstituierte Phenylgruppe oder eine Carboxygruppe repräsentiert.

7. Verbindungen nach einem oder mehreren der Ansprüche 1 bis 6, **dadurch gekennzeichnet, dass** R⁶ ein Wasserstoffatom, ein Halogenatom, eine Hydroxylgruppe, eine lineare oder verzweigte, gesättigte oder ungesättigte, gegebenenfalls mindestens mono-substituierte C₁₋₆-Aliphatengruppe, eine -NH₂-Einheit, eine -CO-NH₂-Einheit, eine -NH-CO-R⁸-Einheit, eine -N(OH)-CO-NH₂-Einheit, eine gegebenenfalls mindestens mono-substituierte Phenylgruppe oder eine Carboxygruppe, bevorzugt ein Wasserstoffatom, eine Hydroxylgruppe oder eine Methylgruppe repräsentiert.

8. Verbindungen nach einem oder mehreren der Ansprüche 1 bis 7, **dadurch gekennzeichnet, dass** R⁷, R⁸, R⁹, R¹⁰ unabhängig voneinander eine lineare oder verzweigte, gesättigte oder ungesättigte, gegebenenfalls mindestens mono-substituierte C₁₋₆-Aliphatengruppe, bevorzugt eine lineare oder verzweigte, gegebenenfalls mindestens mono-substituierte C₁₋₆-Alkylgruppe, bevorzugter eine Methylgruppe oder eine Ethylgruppe, die gegebenenfalls perfluoriert ist, repräsentiert.

9. Verbindungen nach einem oder mehreren der Ansprüche 1 bis 8 der allgemeinen Formel 1 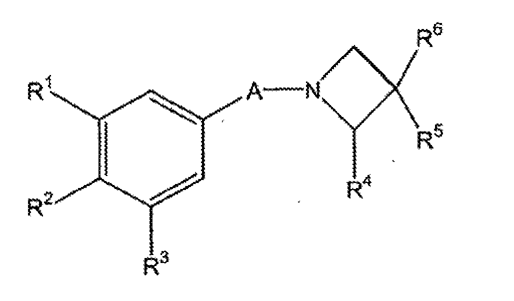 worin
A eine -C=O-Einheit, eine -CH₂-Einheit, eine -CH₂-C=O-Einheit, die über ihr Carbonyl-Kohlenstoffatom an den Azetidin-Ring gebunden ist, oder eine -O-C(=O)-Einheit, die über ihr Carbonyl-Kohlenstoffatom an den Azetidin-Ring gebunden ist, repräsentiert,
R¹, R³ beide gleich sind und eine lineare oder verzweigte C₁₋₄-Alkylgruppe, bevorzugt eine iso-Propylgruppe oder eine tert-Butylgruppe repräsentieren,
R² ein Wasserstoffatom, eine Hydroxylgruppe oder eine lineare oder verzweigte C₁₋₄-Alkoxygruppe repräsentiert,
oder R¹ und R² oder R² und R³ zusammen eine -O-CH₂-C(CH₃)₂-Kette bilden, wobei das Sauerstoffatom der Kette an die 4-Position des Phenylrings gebunden ist,
R⁴ ein Wasserstoffatom, eine lineare oder verzweigte C₁₋₄-Alkylgruppe oder eine unsubstituierte Phenylgruppe repräsentiert,
R⁵ ein Fluoratom, ein Chloratom, ein Bromatom, eine Hydroxylgruppe, eine -NH-CO-CF₃-Einheit, eine -N(OH)-CO-NH₂-Einheit, eine -O(CH₂)₄ONO₂-Einheit oder eine unsubstituierte Phenylgruppe repräsentiert,
R⁶ ein Wasserstoffatom, eine lineare oder verzweigte C₁₋₄-Alkylgruppe oder eine Hydroxylgruppe repräsentiert,
gegebenenfalls in Form eines der Stereoisomere, bevorzugt Enantiomere oder Diastereomere, eines Racemats oder in Form einer Mischung von mindestens zweien der Stereoisomere, bevorzugt Enantiomere und/oder Diastereomere, in jedem Mischungsverhältnis, oder ein korrespondierendes Salz davon oder ein korrespondierendes Solvat davon.

10. Verbindungen nach einem oder mehreren der Ansprüche 1 bis 9 der allgemeinen Formel I 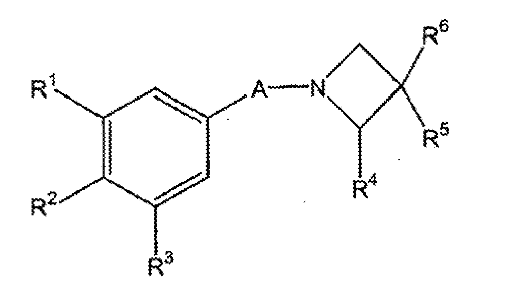 worin
A eine -C=O-Einheit, eine -CH₂-Einheit, eine -CH₂-C=O-Einheit, die über ihr Carbonyl-Kohlenstoffatom an den Azetidin-Ring gebunden ist, oder eine -O-C(=O)-Einheit, die über ihr Carbonyl-Kohlenstoffatom an den Azetidin-Ring gebunden ist, repräsentiert,
R¹, R³ beide gleich sind und eine iso-Propylgruppe oder eine tert-Butylgruppe repräsentieren,
R² ein Wasserstoffatom, eine Hydroxylgruppe oder eine Methoxygruppe repräsentiert,
oder R¹ und R² oder R² und R³ zusammen eine -O-CH₂-C(CH₃)₂-Kette bilden, wobei das Sauerstoffatom der Kette an die 4-Position des Phenylrings gebunden ist,
R⁴ ein Wasserstoffatom, eine Methylgruppe oder eine unsubstituierte Phenylgruppe repräsentiert,
R⁵ ein Bromatom, eine Hydroxylgruppe, eine -NH₂-Einheit, eine -CO-NH₂-Einheit, eine -NH-CO-CF₃-Einheit, eine -N(OH)-CO-NH₂-Einheit, eine -O(CH₂)₄ONO₂-Einheit, eine unsubstituierte Phenylgruppe oder eine Carboxygruppe repräsentiert,
R⁶ ein Wasserstoffatom, eine Methylgruppe oder eine Hydroxylgruppe repräsentiert,
gegebenenfalls in Form eines der Stereoisomere, bevorzugt Enantiomere oder Diastereomere, eines Racemats oder in Form einer Mischung von mindestens zweien der Stereoisomere, bevorzugt Enantiomere und/oder Diastereomere, in jedem Mischungsverhältnis, oder ein korrespondierendes Salz davon, oder ein korrespondierendes Solvat davon.

11. Verbindungen nach einem oder mehreren der Ansprüche 1 bis 10, die ausgewählt sind aus der Gruppe, die besteht aus:
[1] (3,5-Di-tert-butyl-4-hydroxy-phenyl)-(3-hydroxy-azetidin-1-yl)-methanon;
[2] (3,5-Di-tert-butyl-phenyl)-(3-hydroxy-azetidin-1-yl)-methanon;
[3] (3,5-Di-tert-butyl-4-hydroxy-phenyl)-(3-hydroxy-3-methyl-azetidin-1-yl)-methanon;
[4] (3,5-Di-tert-butyl-4-hydroxy-phenyl)-(3-hydroxy-2-methyl-azetidin-1-yl)-methanon;
[7] (3-Brom-azetidin-1-yl)-(3,5-di-tert-butyl-4-hydroxy-phenyl)-methanon;
[9] (3,5-Di-tert-butyl-4-methoxy-phenyl)-(3-hydroxy-azetidin-1-yl)-methanon;
[10] (3-Hydroxy-azetidin-1-yl)-(4-hydroxy-3,5-diisopropyl-phenyl)-methanon,
[11] (3,5-Di-tert-butyl-phenyl)-[3-(4-nitrooxy-butoxy)-azetidin-1-yl]-methanon;
[12] (3,5-Di-tert-butyl-4-hydroxy-phenyl)-(3-hydroxy-2-phenyl-azetidin-1-yl)-methanon;
[13] (3,5-Di-tert-butyl-4-hydroxy-phenyl)-(3-hydroxy-3-phenyl-azetidin-1-yl)-methanon;
[14] (7-Tert-butyl-3,3-dimethyl-2,3-dihydro-benzofuran-5-yl)-(3-hydroxy-azetidin-1-yl)-methanon;
[15] [1-(3,5-Di-tert-butyl-4-hydroxy-benzyl)-azetidin-3-yl]-N-hydroxy-harnstoff;
[16] N-[1-(3,5-Di-tert-butyl-4-hydroxy-benzoyl)-(2S,3R)-2-methyl-azetidin-3-yl]-2,2,2-trifluor-acetamid;
[17] 1-(3,5-Di-tert-butyl-4-hydroxy-benzyl)-azetidin-3-ol;
[18] 2-(3,5-Di-tert-butyl-4-hydroxy-phenyl)-1-(3-hydroxy-azetidin-1-yl)-ethanon;
[19] (3-Hydroxy-azetidin-1-carbonsäure)-3,5-di-tert-butyl-phenylester,
gegebenenfalls in Form eines korrespondierenden Salzes oder eines korrespondierenden Solvats.

12. Verfahren zur Herstellung von substituierten Azetidin-Verbindungen der allgemeinen Formel I gemäß einem oder mehreren der Ansprüche 1 bis 11, **dadurch gekennzeichnet, dass** mindestens eine Verbindung der allgemeinen Formel II 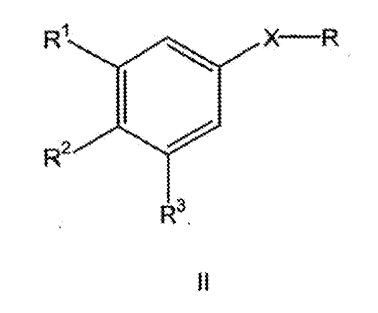 worin R¹ bis R³ die Bedeutung gemäß einem oder mehreren der Ansprüche 1 bis 11 haben, X eine Bindung oder eine -(CH₂)-Einheit repräsentiert, und R eine Carboxygruppe oder eine aktivierte Carbonylgruppe repräsentiert, mit mindestens einer Verbindung der allgemeinen Formel III umgesetzt wird, 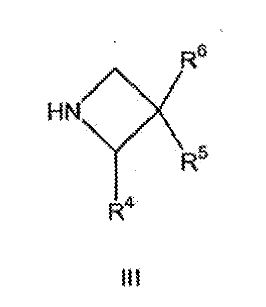 gegebenenfalls in der Form eines korrespondierenden Salzes, worin R⁴ bis R⁶ die Bedeutung gemäß einem oder mehreren der Ansprüche 1 bis 11 haben, um eine Verbindung der allgemeinen Formel I gemäß einem oder mehreren der Ansprüche 1 bis 11 zu ergeben, worin A eine -(C=O)-Einheit oder eine -(CH₂)-CO-Einheit repräsentiert, die gegebenenfalls gereinigt und/oder gegebenenfalls isoliert wird,
und gegebenenfalls mindestens eine Verbindung der allgemeinen Formel I gemäß einem oder mehreren der Ansprüche 1 bis 11, worin A eine -(C=O)-Einheit repräsentiert, wird reduziert, um mindestens eine Verbindung der allgemeinen Formel I gemäß einem oder mehreren der Ansprüche 1 bis 11 zu ergeben, worin A eine -(CH₂)-Einheit repräsentiert, die gegebenenfalls gereinigt und/oder isoliert wird,
oder mindestens eine Verbindung der allgemeinen Formel IV 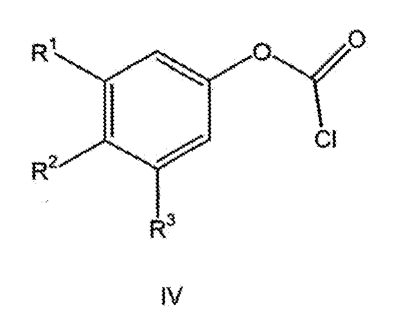 worin R¹ bis R³ die Bedeutung gemäß einem oder mehreren der Ansprüche 1 bis 11 haben, wird mit mindestens einer Verbindung der allgemeinen Formel III, die oben angegeben ist, umgesetzt, um mindestens eine Verbindung der allgemeinen Formel I gemäß einem oder mehreren der Ansprüche 1 bis 10 zu ergeben, worin A eine O-(C=O)-Einheit repräsentiert, und die Verbindung wird gegebenenfalls gereinigt und/oder gegebenenfalls isoliert.

13. Arzneimittel, das mindestens eine substituierte Azetidin-Verbindung gemäß einem oder mehreren der Ansprüche 1 bis 11 und gegebenenfalls einen oder mehrere pharmazeutisch verträgliche Arzneimittelträger umfasst.

14. Arzneimittel nach Anspruch 13 für die Hemmung von Cyclooxygenase-1, für die Prophylaxe und/oder Behandlung von mit Cyclooxygenase-1 zusammenhängenden Erkrankungen, für die Hemmung von Cyclooxygenase-2 und/oder für die Prophylaxe und/der Behandlung von mit Cyclooxygenase-2 zusammenhängenden Erkrankungen.

15. Arzneimittel nach Anspruch 13 oder 14 für die Prophylaxe und/oder Behandlung von Schmerzen, für die Prophylaxe und/oder Behandlung von Entzündungen und/oder für die Prophylaxe und/oder Behandlung von mit Entzündungen zusammenhängenden Erkrankungen, wobei die mit Entzündungen zusammenhängenden Erkrankungen, bevorzugt aus der Gruppe ausgewählt sein können, die aus Arthritis, rheumatoider Arthritis, Spondylarthritis, Gichtarthritis, Gelenkarthrose, systemischem Lupus erythematodes, juveniler Arthritis, rheumatischem Fieber, Symptomen, die mit Grippe oder anderen viralen Infektionen verbunden sind, Erkältung, Schmerzen im unteren Rücken, Nackenschmerzen, Dysmenorrhoe, Kopfschmerzen, Zahnschmerzen, Verstauchungen, Zerrungen, Muskelentzündung, Neuralgie, Synovitis, Gicht, Spondylitis ankylopoetica, Schleimbeutelentzündung, Ödemen, Entzündungen, die Zahnbehandlungen folgen, Entzündungen, die Zahnbehandlungen folgen, Gefäßerkrankungen, Migränekopfschmerzen, Periarteriitis nodosa, Schilddrüsenentzündung, aplastischer Anämie, Morbus Hodgkin, Sklerodermie, Typ-I-Diabetes, dem Erb-Goldflam-Syndrom, Sarkoidose, Nephrose, dem Behcet-Syndrom, Polymyositis, Zahnfleischentzündung, Reizüberempfindlichkeit, Bindehautentzündung, Schwellungen, die nach Verletzungen auftreten, und Herzmuskelischämie besteht, für die Prophylaxe und/oder Behandlung von Asthma, für die Prophylaxe und/oder Behandlung von Bronchitis, für die Prophylaxe und/oder Behandlung von Sehnenentzündungen, für die Prophylaxe und/oder Behandlung von Schleimbeutelentzündungen, für die Prophylaxe und/oder Behandlung von mit der Haut zusammenhängenden Zuständen, wobei die mit der Haut zusammenhängenden Zustände bevorzugt aus der Gruppe ausgewählt sein können, die aus Schuppenflechte, Ekzemen, Verbrennungen und Hautentzündungen besteht, für die Prophylaxe und/oder Behandlung von Magen-Darm-Erkrankungen, wobei die Magen-Darm-Erkrankungen bevorzugt aus der Gruppe ausgewählt sein können, die aus entzündlicher Darmerkrankung, Morbus Crohn, Gastritis, Reizkolon und Colitis ulcerosa besteht, für die Behandlung von Fieber oder für die Prophylaxe und/oder Behandlung von Krebs oder von mit Krebs zusammenhängenden Erkrankungen, wobei der Krebs oder die damit zusammenhängende Erkrankung bevorzugt aus der Gruppe ausgewählt sein kann, die aus Gehirntumor, Knochenkrebs, von Epithelzellen stammende Neoplasie (Epithelkarziom), Basalzellenkarzinom, Adenokarzinom, Magen-Darm-Krebs, Lippenkrebs, Dickdarmkrebs, Leberkrebs, Blasenkrebs, Buchspeicheldrüsenkrebs, Eierstockkrebs, Gebärmutterhalskrebs, Lungenkrebs, Brustkrebs, Hautkrebs, Plattenepithelkarzinom, Prostatakrebs, Nierenzellenkarzinom und anderen bekannten Krebsarten, die Epithelzellen überall im Körper befallen, besteht, für die Prophylaxe und/oder Behandlung von Polypen, für die Prophylaxe und/oder Behandlung von durch Angiogenese vermittelten Erkrankungen, die bevorzugt aus der Gruppe ausgewählt sind, die aus Metastasen, Hornhauttransplantatabstoßung, Okulargefäßneubildung, Netzhautgefäßneubildung, diabetischer Retinopathie, retrolentaler Fibroplasie, neovaskulärem Glaukom, Magengeschwüren, kindlichen Hämangiomen, Angiofibrom des Nasenrachenraums, avaskulärer Nekrose der Knochen und Endometriose besteht.

16. Arzneimittel nach einem oder mehreren der Ansprüche 13 bis 15 für die Prophylaxe und/oder Behandlung von Schmerzen.

17. Arzneimittel nach einem oder mehreren der Ansprüche 13 bis 15 für die Prophylaxe und/oder Behandlung von Entzündungen.

18. Arzneimittel nach einem oder mehreren der Ansprüche 13 bis 15 für die Prophylaxe und/oder Behandlung von mit Entzündungen zusammenhängenden Erkrankungen, wobei die mit Entzündungen zusammenhängenden Erkrankungen bevorzugt aus der Gruppe ausgewählt sein können, die aus Arthritis, rheumatoider Arthritis, Spondylarthritis, Gichtarthritis, Gelenkarthrose, systemischem Lupus erythematodes, juveniler Arthritis, rheumatischem Fieber, Symptomen, die mit Grippe oder anderen viralen Infektionen verbunden sind, Erkältung, Schmerzen im unteren Rücken, Nackenschmerzen, Dysmenorrhoe, Kopfschmerzen, Zahnschmerzen, Verstauchungen, Zerrungen, Muskelentzündung, Neuralgie, Synovitis, Gicht, Spondylitis ankylopoetica, Schleimbeutelentzündung, Ödemen, Entzündungen, die Zahnbehandlungen folgen, Entzündungen, die Zahnbehandlungen folgen, Gefäßerkrankungen, Migränekopfschmerzen, Periarteriitis nodosa, Schilddrüsenentzündung, aplastischer Anämie, Morbus Hodgkin, Sklerodermie, Typ-I-Diabetes, dem Erb-Goldflam-Syndrom, Sarkoidose, Nephrose, dem Behcet-Syndrom, Polymyositis, Zahnfleischentzündung, Reizüberempfindlichkeit, Bindehautentzündung, Schwellungen, die nach Verletzungen auftreten, und Herzmuskelischämie besteht.

19. Verwendung von mindestens einer substituierten Azetidin-Verbindung gemäß einem oder mehreren der Ansprüche 1 bis 11 und gegebenenfalls einem oder mehreren pharmazeutisch verträglichen Arzneimittelträgern für die Herstellung eines Arzneimittels für die Hemmung von Cyclooxygenase-1, für die Prophylaxe und/oder Behandlung von mit Cyclooxygenase-1 zusammenhängenden Erkrankungen, für die Hemmung von Cyclooxygenase-2 und/oder für die Prophylaxe und/oder Behandlung von mit Cyclooxygenase-2 zusammenhängenden Erkrankungen.

20. Verwendung von mindestens einer substituierten Azetidin-Verbindung gemäß einem oder mehreren der Ansprüche 1 bis 11 und gegebenenfalls einem oder mehreren pharmazeutisch verträglichen Arzneimittelträgern für die Herstellung eines Arzneimittels für die Prophylaxe und/oder Behandlung von Schmerzen, für die Prophylaxe und/oder Behandlung von Entzündungen und/oder für die Prophylaxe und/oder Behandlung von mit Entzündungen zusammenhängenden Erkrankungen, wobei die mit Entzündungen zusammenhängenden Erkrankungen, bevorzugt aus der Gruppe ausgewählt sein können, die aus Arthritis, rheumatoider Arthritis, Spondylarthritis, Gichtarthritis, Gelenkarthrose, systemischem Lupus erythematodes, juveniler Arthritis, rheumatischem Fieber, Symptomen, die mit Grippe oder anderen viralen Infektionen verbunden sind, Erkältung, Schmerzen im unteren Rücken, Nackenschmerzen, Dysmenorrhoe, Kopfschmerzen, Zahnschmerzen, Verstauchungen, Zerrungen, Muskelentzündung, Neuralgie, Synovitis, Gicht, Spondylitis ankylopoetica, Schleimbeutelentzündung, Ödemen, Entzündungen, die Zahnbehandlungen folgen, Entzündungen, die Zahnbehandlungen folgen, Gefäßerkrankungen, Migränekopfschmerzen, Periarteriitis nodosa, Schilddrüsenentzündung, aplastischer Anämie, Morbus Hodgkin, Sklerodomie, Typ-I-Diabetes, dem Erb-Goldflam-Syndrom, Sarkoidose, Nephrose, dem Behcet-Syndrom, Polymyositis, Zahnfleischentzündung, Reizüberempfindlichkeit, Bindehautentzündung, Schwellungen, die nach Verletzungen auftreten, und Herzmuskelischämie besteht, für die Prophylaxe und/oder Behandlung von Asthma, für die Prophylaxe und/oder Behandlung von Bronchitis, für die Prophylaxe und/oder Behandlung von Sehnenentzündungen, für die Prophylaxe und/oder Behandlung von Schleimbeutelentzündungen, für die Prophylaxe und/oder Behandlung von mit der Haut zusammenhängenden Zuständen, wobei die mit der Haut zusammenhängenden Zustände bevorzugt aus der Gruppe ausgewählt sein können, die aus Schuppenflechte, Ekzemen, Verbrennungen und Hautentzündungen besteht, für die Prophylaxe und/oder Behandlung von Magen-Darm-Erkrankungen, wobei die Magen-Darm-Erkrankungen bevorzugt aus der Gruppe ausgewählt sein können, die aus entzündlicher Darmerkrankung, Morbus Crohn, Gastritis, Reizkolon und Colitis ulcerosa besteht, für die Behandlung von Fieber oder für die Prophylaxe und/oder Behandlung von Krebs oder von mit Krebs zusammenhängenden Erkrankungen, wobei der Krebs oder die damit zusammenhängende Erkrankung bevorzugt aus der Gruppe ausgewählt sein kann, die aus Gehirntumor, Knochenkrebs, von Epithelzellen stammende Neoplasie (Epithelkarziom), Basalzellkarzinom, Adenokarzinom, Magen-Darm-Krebs, Lippenkrebs, Dickdarmkrebs, Leberkrebs, Blasenkrebs, Buchspeicheldrüsenkrebs, Eierstockkrebs, Gebärmutterhalskrebs, Lungenkrebs, Brustkrebs, Hautkrebs, Plattenepithelkarzinom, Prostatakrebs, Nierenzellenkarzinom und anderen bekannten Krebsarten, die Epithelzellen überall im Körper befallen, besteht, für die Prophylaxe und/oder Behandlung von Polypen, für die Prophylaxe und/oder Behandlung von durch Angiogenese vermittelten Erkrankungen, die bevorzugt aus der Gruppe ausgewählt sind, die aus Metastasen, Hornhauttransplantatabstoßung, Okulargefäßneubildung, Netzhautgefäßneubildung, diabetischer Retinopathie, retrolentaler Fibroplasie, neovaskulärem Glaukom, Magengeschwüren, kindlichen Hämangiomen, Angiofibrom des Nasenrachenraums, avaskulärer Nekrose der Knochen und Endometriose besteht.

21. Verwendung von mindestens einer substituierten Azetidin-Verbindung gemäß einem oder mehreren der Ansprüche 1 bis 11 und gegebenenfalls einem oder mehreren pharmazeutisch verträglichen Arzneimittelträgern für die Herstellung eines Arzneimittels für die Prophylaxe und/oder Behandlung von Schmerzen.

22. Verwendung von mindestens einer substituierten Azetidin-Verbindung gemäß einem oder mehreren der Ansprüche 1 bis 11 und gegebenenfalls einem oder mehreren pharmazeutisch verträglichen Arzneimittelträgern für die Herstellung eines Arzneimittels für die Prophylaxe und/oder Behandlung von Entzündungen.

23. Verwendung von mindestens einer substituierten Azetidin-Verbindung gemäß einem oder mehreren der Ansprüche 1 bis 11 und gegebenenfalls einem oder mehreren pharmazeutisch verträglichen Arzneimittelträgern für die Herstellung eines Arzneimittels für die Prophylaxe und/oder Behandlung von mit Entzündungen zusammenhängenden Erkrankungen, wobei die mit Entzündungen zusammenhängenden Erkrankungen bevorzugt aus der Gruppe ausgewählt sein können, die aus Arthritis, rheumatoider Arthritis, Spondylarthritis, Gichtarthritis, Gelenkarthrose, systemischem Lupus erythematodes, juveniler Arthritis, rheumatischem Fieber, Symptomen, die mit Grippe oder anderen viralen Infektionen verbunden sind, Erkältung, Schmerzen im unteren Rücken, Nackenschmerzen, Dysmenorrhoe, Kopfschmerzen, Zahnschmerzen, Verstauchungen, Zerrungen, Muskelentzündung, Neuralgie, Synovitis, Gicht, Spondylitis ankylopoetica, Schleimbeutelentzündung, Ödemen, Entzündungen, die Zahnbehandlungen folgen, Entzündungen, die Zahnbehandlungen folgen, Gefäßerkrankungen, Migränekopfschmerzen, Periarteriitis nodosa, Schilddrüsenentzündung, aplastischer Anämie, Morbus Hodgkin, Sklerodermie, Typ-I-Diabetes, dem Erb-Goldflam-Syndrom, Sarkoidose, Nephrose, dem Behcet-Syndrom, Polymyositis, Zahnfleischentzündung, Reizüberempfindlichkeit, Bindehautentzündung, Schwellungen, die nach Verletzungen auftreten, und Herzmuskelischämie besteht.

## Revendications

1. Composés azétidine substitués répondant à la formule générale 1, 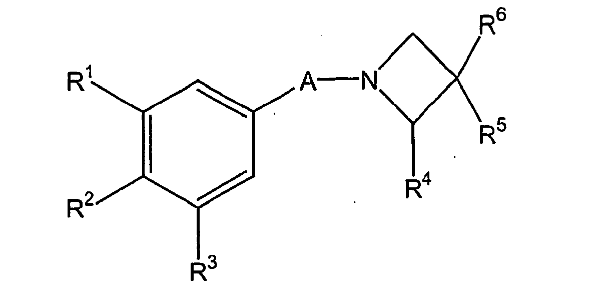 dans laquelle
A représente une entité -C=O, une entité -CH₂, une entité -CH₂-C=O liée au noyau azétidine par l'intermédiaire de son atome de carbone du carbonyle, ou une entité -0-C(=O) liée au noyau azétidine par l'intermédiaire de son atome de carbone du carbonyle,
R¹, R³, identiques ou différents, représentent un atome d'hydrogène ou un groupe aliphatique en C₁ à C₄ saturé ou insaturé, linéaire ou ramifié,
R² représente un atome d'hydrogène, un groupe hydroxy ou un groupe alcoxy en C₁ à C₃,
ou R¹ et R² ou R² et R³ forment ensemble une chaîne -O-CH₂-CH₂, qui est éventuellement substituée par un ou plusieurs groupes méthyle
avec la restriction que R¹, R² et R³ ne représentent pas identiquement un atome d'hydrogène, et que si A représente une entité -CH₂, alors au moins deux des radicaux R¹, R² et R³ ne représentent pas identiquement un atome d'hydrogène,
R⁴ représente un atome d'hydrogène, un groupe aryle éventuellement au moins monosubstitué, un groupe aliphatique saturé ou insaturé, linéaire ou ramifié, qui peut être substitué par un ou plusieurs substituants choisis indépendamment dans le groupe consistant en hydroxy, fluor, chlore, brome, alcoxy en C₁ à C₄ linéaire ou ramifié, perfluoroalcoxy en C₁ à C₄ linéaire ou ramifié et perfluoroalkyle en C₁ à C₄ linéaire ou ramifié,
R⁵ représente un atome d'hydrogène, un atome d'halogène, un groupe hydroxy, un groupe aliphatique saturé ou insaturé, linéaire ou ramifié, éventuellement au moins monosubsituté, une entité -OR⁷, une entité -NH₂, une entité -CO-NH₂, une entité - NH-CO-R⁸, une entité -N(OH)-CO-NH₂, une entité -O(CH₂)₁₋₄-ONO₂, un groupe aryle éventuellement au moins monosubstitué, ou le groupe carboxy,
R⁶ représente un atome d'hydrogène, un atome d'halogène, un groupe hydroxy, un groupe aliphatique saturé ou insaturé, linéaire ou ramifié, éventuellement au moins monosubsituté, une entité -OR⁹, une entité -NH₂, une entité -CO-NH₂, une entité - NH-CO-R¹⁰, une entité -N(OH)-CO-NH₂, un groupe aryle éventuellement au moins monosubstitué, ou le groupe carboxy,
R⁷, R⁸, R⁹, R¹⁰, indépendament l'un de l'autre, représentent un groupe aliphatique saturé ou insaturé, linéaire ou ramifié, éventuellement au moins monosubsituté,
avec les restrictions que
si A représente une entité -(C=O), R⁴ représente un atome d'hydrogène et un des radicaux R et R⁶ représente un atome d'hydrogène, alors l'autre de ces radicaux R⁵ et R⁶ ne représente pas une entité -NH₂, une entité -CONH₂, ou le groupe méthyle, qui est substitué par une entité -NH₂ ou un azahétérocycle, et
si A représente une entité -C=O, une entité -CH₂-C=O liée au noyau azétidine par l'intermédiaire de son atome de carbone du carbonyle, ou une entité -O-C(=O) liée au noyau azétidine par l'intermédiaire de son atome de carbone du carbonyle et un des radicaux R⁵ et R⁶ représente un atome d'hydrogène ou un groupe aliphatique saturé ou insaturé, linéaire ou ramifié, éventuellement au moins monosubsituté, alors l'autre de ces radicaux R⁵ et R⁶ ne représente pas une entité -NH₂ ou une entité -COOH,
éventuellement sous forme d'un des stéréoisomères, de préférence énantiomères ou diastéréoisomères, d'un racémate ou sous forme d'un mélange d'au moins deux des stéréoisomères, de préférence énantiomères et/ou diastéréoisomères, selon un quelconque rapport de mélange, ou un sel correspondant de ceux-ci, ou un solvate correspondant de ceux-ci.

2. Composés selon la revendication 1, **caractérisés en ce que** R¹ et R³, identiques ou différents, représentent l'atome d'hydrogène ou un groupe alkyle en C₁ à C₄ linéaire ou ramifié.

3. Composés selon la revendication 1 ou 2, **caractérisés en ce que** R¹ et R³ sont identiques et représentent un groupe alkyle en C₁ à C₄, de préférence un groupe alkyle en C₃ à C₄, de préférence encore un groupe isopropyle ou un groupe tert-butyle.

4. Composés selon une ou plusieurs des revendications 1 à 3, **caractérisés en ce que** R² représente un atome d'hydrogène, un groupe hydroxy ou un groupe méthoxy.

5. Composés selon une ou plusieurs des revendications 1 à 4, **caractérisés en ce que** R⁴ représente un atome d'hydrogène, un groupe phényle éventuellement au moins monosubstitué, ou un groupe aliphatique en C₁ à C₆ saturé ou insaturé, linéaire ou ramifié, ledit groupe aliphatique pouvant être substitué par un ou plusieurs substituants indépendamment choisis dans le groupe consistant en hydroxy, fluor, chlore, brome, alcoxy en C₁ à C₄ linéaire ou ramifié, perfluoroalcoxy en C₁ à C₄ linéaire ou ramifié et perfluoroalkyle en C₁ à C₄ linéaire ou ramifié, de préférence un atome d'hydrogène, un groupe méthyle ou un groupe phényle non substitué.

6. Composés selon une ou plusieurs des revendications 1 à 5, **caractérisés en ce que** R⁵ représente un atome d'hydrogène, un atome d'halogène, un groupe hydroxy, un groupe aliphatique en C₁ à C₆ saturé ou insaturé, linéaire ou ramifié, éventuellement au moins monosubstitué, une entité -NH₂, une entité -CO-NH₂, une entité -NH-CO-R⁸, une entité -N(OH)-CO-NH₂, une entité -O(CH₂)₄-ONO₂, un groupe phényle éventuellement au moins monosubstitué, ou un groupe carboxy, de préférence un atome d'hydrogène, un atome de brome, un groupe hydroxy, une entité -NH₂, une entité -CO-NH₂, une entité -NH-CO-R⁸, une entité -N(OH)-CO-NH₂, une entité -O(CH₂)₄-ONO₂, un groupe phényle non substitué, ou un groupe carboxy.

7. Composés selon une ou plusieurs des revendications 1 à 6, **caractérisés en ce que** R⁶ représente unatome d'hydrogène, un atome d'halogène, un groupe hydroxy, un groupe aliphatique en C₁ à C₆ saturé ou insaturé, linéaire ou ramifié, éventuellement au moins monosubstitué, une entité -NH₂, une entité -CO-NH₂, une entité -NH-COR⁸, une entité -N(OH)-CO-NH₂, un groupe phényle éventuellement au moins monosubstitué, ou un groupe carboxy, de préférence un atome d'hydrogène, un groupe hydroxy ou un groupe méthyle.

8. Composés selon une ou plusieurs des revendications 1 à 7, **caractérisés en ce que** R⁷, R⁸, R⁹, R¹⁰, indépendamment fun de l'autre, représentent un groupe aliphatique en C₁ à C₆ saturé ou insaturé, linéaire ou ramifié, de préférence un groupe alkyle en C₁ à C₆ linéaire ou ramifié, éventuellement au moins monosubstitué, de préférence encore un groupe méthyle ou un groupe éthyle, qui est éventuellement perfluoré.

9. Composés selon une ou plusieurs des revendications 1 à 8 répondant à la formule générale 1, 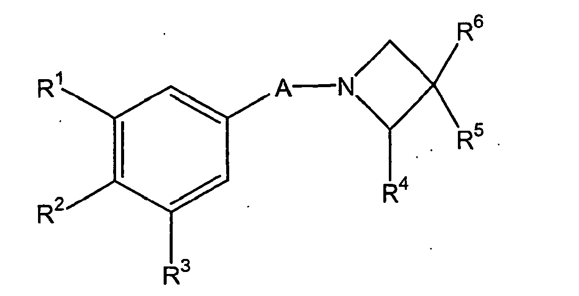 dans laquelle
A représente une entité -C=O, une entité -CH₂, une entité -CH₂-C=O liée au noyau azétidine par l'intermédiaire de son atome de carbone du carbonyle, ou une entité -OC(=O)- liée au noyau azétidine par l'intermédiaire de son atome de carbone du carbonyle,
R¹, R³ représentent tous les deux identiquement un groupe alkyle en C₁ à C₄ linéaire ou ramifié, de préférence un groupe isopropyle ou un groupe tert-butyle,
R² représente un atome d'hydrogène, un groupe hydroxy ou un groupe alcoxy en C₁ à C₄ linéaire ou ramifié,
ou R¹ et R² ou R² et R³ forment ensemble une chaîne -O-CH₂-C(CH₃)₂, l'atome d'oxygène de ladite chaîne étant lié en position 4 du noyau phényle,
R⁴ représente un atome d'hydrogène, un groupe alkyle en C₁ à C₄ linéaire ou ramifié ou le groupe phényle non substitué,
R⁵ représente un atome de fluor, un atome de chlore, un atome de brome, un groupe hydroxy, une entité -NH-CO-CF₃, une entité -N(OH)-CO-NH₂, une entité -O-(CH₂)₄ONO₂, ou un groupe phényle non substitué,
R⁶ représente un atome d'hydrogène, un groupe alkyle en C₁ à C₄ linéaire ou ramifié, ou un groupe hydroxy,
éventuellement sous forme d'un des stéréoisomères, de préférence énantiomères ou diastéréoisomères, d'un racémate ou sous forme d'un mélange d'au moins deux des stéréoisomères, de préférence énantiomères et/ou diastéréoisomères, selon un quelconque rapport de mélange, ou un sel correspondant de ceux-ci, ou un solvate correspondant de ceux-ci.

10. Composés selon une ou plusieurs des revendications 1 à 9 répondant à la formule générale I, 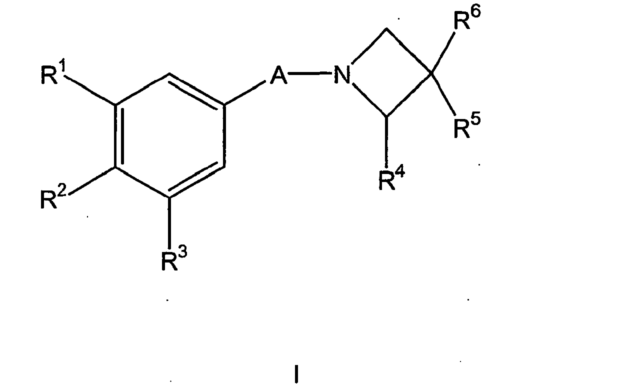 dans laquelle
A représente une entité -C=O, une entité -CH₂, une entité -CH₂-C=O liée au noyau azétidine par l'intermédiaire de son atome de carbone du carbonyle, ou une entité -OC(=O) liée au noyau azétidine par l'intermédiaire de son atome de carbone du carbonyle,
R¹, R³ représentent tous les deux identiquement un groupe isopropyle ou un groupe tert-butyle,
R² représente un atome d'hydrogène, un groupe hydroxy ou un groupe méthoxy,
ou R¹ et R² ou R² et R³ forment ensemble une chaîne -O-CH₂-C(CH₃)₂, l'atome d'oxygène de ladite chaîne étant lié en position 4 du noyau phényle,
R⁴ représente un atome d'hydrogène, un groupe méthyle ou un groupe phényle non substitué,
R⁵ représente un atome de brome, un groupe hydroxy, une entité -NH₂, une entité - CO-NH₂, une entité -NH-CO-CF₃, une entité -N(OH)-CO-NH₂, une entité -O-(CH₂)₄ONO₂, le groupe phényle non substitué, ou le groupe carboxy,
R⁶ représentent unatome d'hydrogène, un groupe méthyle ou un groupe hydroxy, éventuellement sous forme d'un des stéréoisomères, de préférence énantiomères ou diastéréoisomères, d'un racémate ou sous forme d'un mélange d'au moins deux des stéréoisomères, de préférence énantiomères et/ou diastéréoisomères, selon un quelconque rapport de mélange, ou un sel correspondant de ceux-ci, ou un solvate correspondant de ceux-ci.

11. Composés selon une ou plusieurs des revendications 1 à 10 choisis dans le groupe consistant en
[1] (3,5-di-tert-butyl-4-hydroxy-phényl)-(3-hydroxy-azétidin-1-yl)-méthanone ;
[2] (3,5-di-tert-butyl-phényl)-(3-hydroxy-azétidin-1-yl)-méthanone ;
[3] (3,5-di-tert-butyl-4-hydroxy-phényl)-(3-hydroxy-3-méthyl-azétidin-1-yl)-méthanone ;
[4] (3,5-di-tert-butyl-4-hydroxy-phényl)-(3-hydroxy-2-méthyl-azétidin-1-yl)-méthanone ;
[7] (3-bromo-azétidin-1-yl)-(3,5-di-tert-butyl-4-hydroxy-phényl)-méthanone ;
[9] (3,5-di-tert-butyl-4-méthoxy-phényl)-(3-hydroxy-azétidin-1-yl)-méthanone ;
[10] (3-hydroxy-azétidin-1-yl)-(4-hydroxy-3,5-düsopropyl-phényl)-méthanone ;
[11] (3,5-di-tert-butyl-phényl)-[3-(4-nitrooxy-butoxy)-azétidin-1-yl]-méthanone ;
[12] (3,5-di-tert-butyl-4-hydroxy-phényl)-(3-hydroxy-2-phényl-azétidin-1-yl)-méthanone ;
[13] (3,5 -di-tert-butyl-4-hydroxy-phényl)-(3 -hydroxy-3 -phényl-azétidin-1 -yl)-méthanone ;
[14] (7-tert-butyl-3,3-diméthyl-2,3-dihydro-benzofuran-5-yl)-(3-hydroxy-azétidin-1-yl)-méthanone ;
[15] [1-(3,5-di-tert-butyl-4-hydroxy-benzyl)-azétidin-3-yl]-N-hydroxy-urée ;
[16] N-[1-(3,5-di-tert-butyl-4-hydroxy-benzoyl)-(2S,3R)-2-méthyl-azétidin-3-yl]-2,2,2-trifluoro-acétamide ;
[17] 1-(3,5-di-tert-butyl-4-hydroxy-benzyl)-azétidin-3-ol ,
[18] 2-(3,5-di-tert-butyl-4-hydroxy-phényl)-1-(3-hydroxy-azétidin-1-yl)-éthanone ;
[19] ester 3,5-di-tert-butyl-phénylique de l'acide 3-hydroxy-azétidine-1-carboxylique
éventuellement sous forme d'un sel correspondant ou d'un solvate correspondant.

12. Procédé de préparation de composés azétidine substitués de formule générale I selon une ou plusieurs des revendications 1 à 11, **caractérisé en ce que** au moins un composé répondant à la formule générale II, 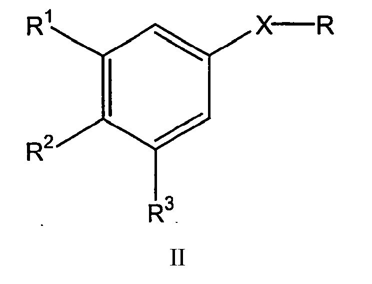 dans laquelle R¹ à R³ ont la signification donnée selon une ou plusieurs des revendications 1 à 11, X représente une liaison ou une entité -(CH₂)- et R représente le groupe carboxy ou un groupe carbonyle activé, est mis en réaction avec au moins un composé répondant à la formule III, 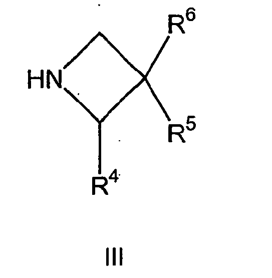 éventuellement sous forme d'un sel correspondant, dans laquelle R⁴ à R⁶ ont la signification donnée selon une ou plusieurs des revendications 1 à 11, pour donner un composé répondant à la formule générale I selon une ou plusieurs des revendications 1 à 11, dans laquelle A représente une entité -(C=O) ou une entité - (CH₂)-CO, qui est éventuellement purifié et/ou éventuellement isolé,
et éventuellement au moins un composé répondant à la formule générale I selon une ou plusieurs des revendications 1 à 11, dans laquelle A représente une entité -(C=O) est réduit pour donner au moins un composé répondant à la formule générale I selon une ou plusieurs des revendications 1 à 11, dans laquelle A représente une entité - (CH₂), qui est éventuellement purifié et/ou éventuellement isolé,
ou au moins un composé répondant à la formule générale IV, 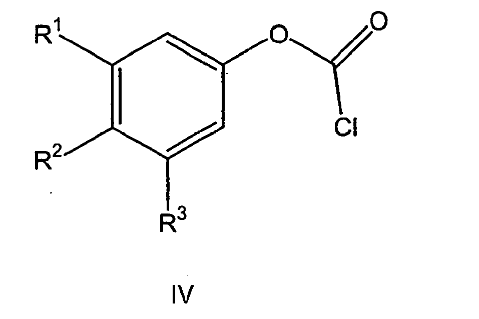 dans laquelle R¹ à R³ ont la signification donnée selon une ou plusieurs des revendications 1 à 11, est mis en réaction avec au moins un composé répondant à la formule générale III donnée ci-dessus, pour donner au moins un composé répondant à la formule générale I selon une ou plusieurs des revendications 1 à 10, dans laquelle A représente une entité -O-(C=O), et ledit composé est éventuellement purifié et/ou éventuellement isolé.

13. Médicament comprenant au moins un composé azétidine substitué selon une ou plusieurs des revendications 1 à 11 et éventuellement un ou plusieurs excipients acceptables du point de vue pharmaceutique.

14. Médicament selon la revendication 13 pour l'inhibition de la cyclooxygénase 1, pour la prophylaxie et/ou le traitement de troubles associés à la cyclooxygénase 1, pour l'inhibition de la cyclooxgénase 2 et/ou pour la prophylaxie et/ou le traitement de troubles associés à la cyclooxygénase 2.

15. Médicament selon la revendication 13 ou 14 pour la prophylaxie et/ou le traitement d'une douleur, pour la prophylaxie et/ou le traitement d'une inflammation et/ou pour la prophylaxie et/ou le traitement de troubles associés à une inflammation, lesdits troubles associés à une inflammation pouvant de préférence être choisis dans le groupe consistant en l'arthrite, l'arthrite rhumatoïde, des spondyloarthropathies, l'arthrite goutteuse, l'ostéoarthrite, le lupus érythémateux disséminé, l'arthrite juvénile, la fièvre rhumatoïde, des symptômes associés à la grippe ou à d'autres infections virales, le rhume commun, une douleur lombaire basse, une douleur cervicale, la dysménorrhée, les maux de tête, les douleurs dentaires, les entorses, les froissements musculaires, la myosite, la névralgie, la synovite, la goutte, la spondylite ankylosante, la bursite, un oedème, des inflammations à la suite d'interventions dentaires, des inflammations à la suite d'interventions dentaires, des maladies vasculaires, les maux de tête migraineux, la périartérite noueuse, la thyroïdite, l'anémie aplastique, la maladie de Hodkin, la sclérodermie, le diabète de type I, la myasthénie intense, la sarcoïdose, le syndrome néphrotique, le syndrome de Behcet, la polymyosite, la gingivite, l'hypersensibilité, la conjunctivite, une enflure à la suite d'une blessure et l'ischémie du myocarde, pour la prophylaxie et/ou le traitement de l'asthme, pour la prophylaxie et/ou le traitement de la bronchite, pour la prophylaxie et/ou le traitement de la tendinite, pour la prophylaxie et/ou le traitement de la bursite, pour la prophylaxie et/ou le traitement d'un état associé à la peau, ledit état associé à la peau pouvant de préférence être choisi dans le groupe consistant en le psoriasis, l'eczéma, les brûlures et la dermatite, pour la prophylaxie et/ou le traitement de troubles gastro-intestinaux, lesdits troubles gastro-intestinaux pouvant de préférence être choisis dans le groupe consistant en les troubles intestinaux inflammatoires, la maladie de Crohn, la gastrite, le syndrome de l'intestin irritable et la colite ulcérative, ou pour le traitement de la fièvre, ou pour la prophylaxie et/ou le traitement du cancer ou de troubles associés au cancer, ledit cancer ou trouble associé pouvant de préférence être choisi dans le groupe consistant en le cancer du cerveau, le cancer des os, la néoplasie associée aux cellules épithéliales (carcinome épithélial), le carcinome des cellules basales, l'adénocarcinome, le cancer gastro-intestinal, le cancer des lèvres, le cancer du colon, le cancer du foie, le cancer de la vessie, le cancer du pancréas, le cancer des ovaires, le cancer du col de l'utérus, le cancer du poumon, le cancer du sein, le cancer de la peau, le cancer des cellules squameuses, le cancer de la prostate, le carcinome des cellules rénales et d'autres cancers connus qui affectent les cellules épithéliales dans tout le corps, pour la prophylaxie et/ou le traitement de polypes, pour la prophylaxie et/ou le traitement de troubles associés à fangiogenèse, de préférence choisis dans le groupe consistant en la métastase, le rejet de greffe de cornée, la néovascularisation oculaire, la néovascularisation rétinale, la rétinopathie diabétique, la fibroplasie rétrolentale, le glaucome néovasculaire, l'ulcère gastrique, l'hémaginome de l'enfant, l'angiofibrome du nasopharynx, la nécrose avasculaire des os et l'endométriose.

16. Médicament selon une ou plusieurs des revendications 13 à 15 pour la prophylaxie et/ou le traitement d'une douleur.

17. Médicament selon une ou plusieurs des revendications 13 à 15 pour la prophylaxie et/ou le traitement d'une inflammation.

18. Médicament selon une ou plusieurs des revendications 13 à 15 pour la prophylaxie et/ou le traitement de troubles associés à une inflammation, lesdits troubles associés à une inflammation pouvant de préférence être choisis dans le groupe consistant en l'arthrite, l'arthrite rhumatoïde, des spondyloarthropathies, l'arthrite goutteuse, l'ostéoarthrite, le lupus érythémateux disséminé, l'arthrite juvénile, la fièvre rhumatoïde, des symptômes associés à la grippe ou à d'autres infections virales, le rhume commun, une douleur lombaire basse, une douleur cervicale, la dysménorrhée, les maux de tête, les douleurs dentaires, les entorses, les froissements musculaires, la myosite, la névralgie, la synovite, la goutte, la spondylite ankylosante, la bursite, un oedème, des inflammations à la suite d'interventions dentaires, des inflammations à la suite d'interventions dentaires, des maladies vasculaires, les maux de tête migraineux, la périartérite noueuse, la thyroïdite, l'anémie aplastique, la maladie de Hodkin, la sclérodermie, le diabète de type I, la myasthénie intense, la sarcoïdose, le syndrome néphrotique, le syndrome de Behcet, la polymyosite, la gingivite, l'hypersensibilité, la conjunctivite, une enflure à la suite d'une blessure et l'ischémie du myocarde.

19. Utilisation d'au moins un composé azétidine substitué selon une ou plusieurs des revendications 1 à 11 et éventuellement d'un ou plusieurs excipients acceptables du point de vue pharmaceutique, pour la préparation d'un médicament pour l'inhibition de la cyclooxygénase 1, pour la prophylaxie et/ou le traitement de troubles associés à la cyclooxygénase l, pour l'inhibition de la cyclooxgénase 2 et/ou pour la prophylaxie et/ou le traitement de troubles associés à la cyclooxygénase 2.

20. Utilisation d'au moins un composé azétidine substitué selon une ou plusieurs des revendications 1 à 11 et éventuellement d'un ou plusieurs excipients acceptables du point de vue pharmaceutique, pour la préparation d'un médicament pour la prophylaxie et/ou le traitement d'une douleur, pour la prophylaxie et/ou le traitement d'une inflammation et/ou pour la prophylaxie et/ou le traitement de troubles associés à une inflammation, lesdits troubles associés à une inflammation pouvant de préférence être choisis dans le groupe consistant en l'arthrite, l'arthrite rhumatoïde, des spondyloarthropathies, l'arthrite goutteuse, l'ostéoarthrite, le lupus érythémateux disséminé, l'arthrite juvénile, la fièvre rhumatoïde, des symptômes associés à la grippe ou à d'autres infections virales, le rhume commun, une douleur lombaire basse, une douleur cervicale, la dysménorrhée, les maux de tête, les douleurs dentaires, les entorses, les froissements musculaires, la myosite, la névralgie, la synovite, la goutte, la spondylite ankylosante, la bursite, un oedème, des inflammations à la suite d'interventions dentaires, des inflammations à la suite d'interventions dentaires, des maladies vasculaires, les maux de tête migraineux, la périartérite noueuse, la thyroïdite, l'anémie aplastique, la maladie de Hodkin, la sclérodermie, le diabète de type I, la myasthénie intense, la sarcoïdose, le syndrome néphrotique, le syndrome de Behcet, la polymyosite, la gingivite, l'hypersensibilité, la conjunctivite, une enflure à la suite d'une blessure et l'ischémie du myocarde, pour la prophylaxie et/ou le traitement de l'asthme, pour la prophylaxie et/ou le traitement de la bronchite, pour la prophylaxie et/ou le traitement de la tendinite, pour la prophylaxie et/ou le traitement de la bursite, pour la prophylaxie et/ou le traitement d'un état associé à la peau, ledit état associé à la peau pouvant de préférence être choisi dans le groupe consistant en le psoriasis, l'eczéma, les brûlures et la dermatite, pour la prophylaxie et/ou le traitement de troubles gastro-intestinaux, lesdits troubles gastro-intestinaux pouvant de préférence être choisis dans le groupe consistant en les troubles intestinaux inflammatoires, la maladie de Crohn, la gastrite, le syndrome de l'intestin irritable et la colite ulcérative, ou pour le traitement de la fièvre, ou pour la prophylaxie et/ou le traitement du cancer ou de troubles associés au cancer, ledit cancer ou trouble associé pouvant de préférence être choisi dans le groupe consistant en le cancer du cerveau, le cancer des os, la néoplasie associée aux cellules épithéliales (carcinome épithélial), le carcinome des cellules basales, l'adénocarcinome, le cancer gastro-intestinal, le cancer des lèvres, le cancer du colon, le cancer du foie, le cancer de la vessie, le cancer du pancréas, le cancer des ovaires, le cancer du col de l'utérus, le cancer du poumon, le cancer du sein, le cancer de la peau, le cancer des cellules squameuses, le cancer de la prostate, le carcinome des cellules rénales et d'autres cancers connus qui affectent les cellules épithéliales dans tout le corps, pour la prophylaxie et/ou le traitement de polypes, pour la prophylaxie et/ou le traitement de troubles associés à l'angiogenèse, de préférence choisis dans le groupe consistant en la métastase, le rejet de greffe de cornée, la néovascularisation oculaire, la néovascularisation rétinale, la rétinopathie diabétique, la fibroplasie rétrolentale, le glaucome néovasculaire, l'ulcère gastrique, l'hémaginome de l'enfant, l'angiofibrome du nasopharynx, la nécrose avasculaire des os et l'endométriose.

21. Utilisation d'au moins un composé azétidine substitué selon une ou plusieurs des revendications 1 à 11 et éventuellement d'un ou plusieurs excipients acceptables du point de vue pharmaceutique, pour la préparation d'un médicament pour la prophylaxie et/ou le traitement d'une douleur.

22. Utilisation d'au moins un composé azétidine substitué selon une ou plusieurs des revendications 1 à 11 et éventuellement d'un ou plusieurs excipients acceptables du point de vue pharmaceutique, pour la préparation d'un médicament pour la prophylaxie et/ou le traitement d'une inflammation.

23. Utilisation d'au moins un composé azétidine substitué selon une ou plusieurs des revendications 1 à 11 et éventuellement d'un ou plusieurs excipients acceptables du point de vue pharmaceutique, pour la préparation d'un médicament pour la prophylaxie et/ou le traitement de troubles associés à une inflammation, lesdits troubles associés à une inflammation pouvant de préférence être choisis dans le groupe consistant en l'arthrite, l'arthrite rhumatoïde, des spondyloarthropathies, l'arthrite goutteuse, l'ostéoarthrite, le lupus érythémateux disséminé, l'arthrite juvénile, la fièvre rhumatoïde, des symptômes associés à la grippe ou à d'autres infections virales, le rhume commun, une douleur lombaire basse, une douleur cervicale, la dysménorrhée, les maux de tête, les douleurs dentaires, les entorses, les froissements musculaires, la myosite, la névralgie, la synovite, la goutte, la spondylite ankylosante, la bursite, un oedème, des inflammations à la suite d'interventions dentaires, des inflammations à la suite d'interventions dentaires, des maladies vasculaires, les maux de tête migraineux, la périartérite noueuse, la thyroïdite, l'anémie aplastique, la maladie de Hodkin, la sclérodermie, le diabète de type I, la myasthénie intense, la sarcoïdose, le syndrome néphrotique, le syndrome de Behcet, la polymyosite, la gingivite, l'hypersensibilité, la conjunctivite, une enflure à la suite d'une blessure et l'ischémie du myocarde.
